# FASCICULE DE BREVET EUROPEEN

(11) **EP 1 047 299 B1**
(45) Date de publication et mention de la délivrance du brevet: **19.06.2002**
(21) Numéro de dépôt: 99954071.9
(22) Date de dépôt: 05.11.1999
(51) Int. Cl.: A01N 1/02

(54) **SOLUTION DE PERFUSION ET/OU DE CONSERVATION ET/OU DE REPERFUSION LORS DE LA TRANSPLANTATION D'ORGANES**
PERFUSIONS- UND/ODER KONSERVIERUNGS- UND/ODER REPERFUSIONSLÖSUNG ZUR ORGANTRANSPLANTATION
PERFUSION AND/OR PRESERVATION AND/OR RE-PERFUSION SOLUTION DURING ORGAN TRANSPLANT

(30) Priorité: 10.11.1998 FR 9814132
(43) Date de publication de la demande: 02.11.2000
(73) Titulaire: CENTRE NATIONAL DE LA RECHERCHE SCIENTIFIQUE (CNRS), 75794 Paris Cedex 16 (FR)
(72) Inventeur: COZZONE, Patrick, 13007 Marseille (FR); BERNARD, Monique, La Boiseraie, 13012 Marseille (FR)
(74) Mandataire: Grosset-Fournier, Chantal Catherine
(86) Numéro de dépôt international: FR9902711
(87) Numéro de publication internationale: WO0027189

(56) Documents cités:
- FR-A- 2 695 827
- US-A- 5 290 766
- US-A- 5 407 793
- E. KEVELAITIS ET AL.: "Poststorage diastolic abnormalities of heart transplants: is vascular dysfunction or myocardial contracture the culprit?" THE JOURNAL OF HEART AND LUNG TRANSPLANTATION, vol. 15, no. 5, 1996, pages 461-469, XP002110980

## Description

L'invention a pour objet une solution de perfusion et/ou de conservation et/ou de reperfusion lors de la transplantation d'organes, notamment de l'organe cardiaque.

La période de préservation des coeurs humains est à présent de 4 heures et un certain nombre de rejets sont encore dus à une altération de l'état du greffon entre la période de prélèvement et le moment de l'implantation chez le receveur.

La préservation myocardique à court terme (4 heures) est assurée actuellement par une conservation au froid après un arrêt cardioplégique. Il existe cependant une variété de procédés différant par la composition de la solution utilisée, la température de la conservation et le protocole d'administration. Différentes solutions d'arrêt et de préservation du coeur ont été développées pour protéger le myocarde en chirurgie cardiaque.

La solution de St Thomas [Ledingham, S.J.M., Braimbridge, M.V., Hearse, D.J. The St Thomas' hospital cardioplegic solution: a comparison of the efficacay of two formulations. *J. Thorac. Cardiovasc. Surg.* (1987) 93: 240-246] a été en particulier très utilisée.

Des solutions proches de la solution de St Thomas ont été développées telles que la solution de Broussais [Fabiani, J.N., Ponzio, O., Jebara, V. La protection myocardique; encycl. Méd. Chir. (Paris, France)., Techniques chirurgicales, Thorax, 42511, 10-1989].

Plus récemment des expériences ont été réalisées avec la solution de l'Université de Wisconsin (UW) [Ledingham, S.J.M., Katayama, O., Lachno, D.R., Yacoub, M.H. Prolonged cardiac preservation. Evaluation of the University of Wisconsin preservation solution by comparison with the St Thomas' solution in the rat. *Circulation* (1990) 82 (Part2): IV351-8] couramment utilisée pour d'autres organes (foie, rein). L'utilisation de la solution UW a permis en matière de transplantation rénale et hépatique, des progrès impressionnants dans la conservation du greffon en augmentant significativement la durée d'ischémie froide avant transplantation.

Certaines équipes ont déjà fait état de résultats intéressants en appliquant la solution UW à la conservation du greffon cardiaque. Cette solution comporte différents éléments protecteurs ; le glutathion et l'allopurinol, inhibiteurs de la formation des radicaux libres de l'oxygène, des agents imperméants (lactobionate et raffinose) et de l'adénosine, un précurseur de l'ATP.

Différentes solutions dérivées directement de la solution UW ont été développées, d'autres encore ne reprennent que certains éléments de la solution UW (solution Celsior) [Ménasché, P., Termignon, JL., Pradier, F., Grousset, C., Mouas, C. Expérimental evaluation of Celsior, a new heart preservation solution. *Eur J. Cardio. Thorac. Surg.* (1994) 8 : 207-213].

Il a été montré que la solution de l'Université de Wisconsin (UW) permettait d'améliorer la durée de préservation du myocarde. Cette solution comporte différents éléments protecteurs, le glutathion et l'allopurinol, inhibiteurs de la formation des radicaux libres de l'oxygène, des agents imperméants (lactobionate, raffinose), un précurseur de l'ATP (adénosine). Par contre, cette solution n'a pas été développée spécifiquement pour le coeur et présente différentes caractéristiques qui ne sont pas optimales sinon dommageables pour le coeur: 1) la solution UW est caractérisée par exemple par une forte concentration en K⁺ (125 mM) dommageable pour la cellule car elle entraîne une contracture, 2) le phosphate inorganique (Pi) est également présent à forte concentration or celui-ci est un inhibiteur de nombreuses ATPases (Na⁺/K⁺ ATPase, Ca2⁺ ATPases) et peut ainsi accroître l'accumulation en Na⁺ et Ca2⁺ délétère en cours de l'ischémie, 3) elle ne comprend pas de Ca²⁺ et de ce fait induit un influx massif de Ca²⁺ lors de la reperfusion, 4) l'adénosine a de nombreux effets dans le coeur et sa concentration n'est pas optimisée dans la solution, 5) la solution UW ne comprend pas un certain nombre d'éléments dont l'effet protecteur a pu être démontré dans le coeur.

La référence Kevelaitis et al. (Journal of Heart and Lung Transplantation 1996; 15: 461-469) décrit une nouvelle solution de conservation du coeur, constituée d'une solution de Celsior, complémentée en L-arginine et en 2,3-butanedione monoxime. La solution de Celsior utilisée a la composition suivante (mmol/L) : K+, 15 ; sodium, 100 ; magnésium, 13 ; calcium, 0,26 ; chlorure, 41,5 ; histidine, 30 ; glutamate, 20 ; lactobionate, 80 ; mannitol, 60 ; pH 7,3 à 7,4 à 20°C ; osmolarité (mOsm/L), 360.

Cette solution ne contient ni adénosine, ni allopurinol et ni raffinose, mais contient du 2, 3-butanedione monoxime et de l'histidine.

Les résultats sont donnés après 10 heures de conversation mais rien n'est indiqué pour des périodes de conservation plus longues.

Le brevet français n°FR 2 695 827, décrit une solution unique de perfusion, de conservation et de reperfusion d'organes.

Cette solution contient de l'histidine, mais ne contient ni arginine, ni adénosine, ni allopurinol et ni raffinose.

Par ailleurs, la durée de conservation n'est pas étudiée.

Le brevet américain n° US 5 407 793 décrit à la fois un procédé et une solution aqueuse cardioplégique pour la chirurgie cardiaque et, un procédé et une solution aqueuse de conservation du coeur pour la transplantation. Une solution aqueuse de conservation du coeur présente la composition suivante (mmol/L : Histidine, 50 à 150 ; glucose, 11 ; Na⁺, 20 à 120 ; K⁺, 5 à 30 ; Mg²⁺, 4 à 16 ; Ca²⁺, 0,05 à 1 ; insuline : 1 à 40 UI/L ; adénosine, 1 à 10 ; lidocaïne, 100mg/L. ; PO₄³⁻ 1,25 à 3 ; mannitol, 10 à 20. Une solution acqueuse cardioplégique présente la même composition que celle de la solution décrite précédemment, la seule différence résidant dans la teneur en adénosine qui varie d'environ 0,001 à 1 mmol/L.

Cette solution ne contient ni glutamate, ni arginine, mais contient de la lidocaïne. Par ailleurs, les protocoles permettant des durées de préservation les plus longues sont réalisés avec des perfusions multiples de la solution au cours de la période d'ischémie, procédé qui est lourd à mettre en oeuvre lors du transport d'organes.

Le brevet américain n° US 5 290 766 a pour objet une solution stérile aqueuse cardioplégique comprenant : (mmoles/L) NaCl, 70 à 11 ; KCl, 10 à 16 ; CaCl₂, 0,5 à 1 ; MgCl₂, 8 à 16 ; NaHCO₃, 10 à 20 ; D-glucose, 5 à 15, acide L-aspartique, 5 à 20 ; acide lactobionique, 0 à 15.

Cette solution ne contient ni glutamate, ni arginine, ni adénosine, ni glutathion, ni allopurinol. La récupération du coeur est étudiée après une ischémie de 6 heures, mais il n'a pas été mis en évidence une bonne préservation après une durée plus longue.

Il y a plusieurs années une solution de cardioplégie (solution B20) a été développée pour des durées d'ischémie courtes (2 à 3 heures) [Monique Bernard et al. J. Thorac. Cardiovasc Surg 90 : 235-242, 1985].

Si l'on considère les besoins exprimés par les chirurgiens, seules les options d'un allongement de la durée de préservation à 12 heures ou à 6 mois ou plus présentent un intérêt. Un passage de 4 à 12 heures de préservation correspond en effet beaucoup plus qu'à un incrément marginal de la durée d'ischémie. Il présente plusieurs avantages ; (1) une extension de la zone géographique dans laquelle la collecte des greffons est pratiquée et donc une augmentation pour un patient donné des chances d'être greffé en lui offrant la possibilité d'utiliser un greffon prélevé, géographiquement à distance plus éloignée, (2) de pouvoir envisager de greffer en compatibilité HLA en dégageant le temps nécessaire pour effectuer le meilleur appariement possible donneur-receveur (de telles greffes ont déjà montré en matière de transplantation rénale de bien meilleurs résultats en terme de survie du greffon, que le simple appariement ABO actuellement utilisé pour les greffes cardiaques), (3) enfin, le passage de 4 à 12 heures laisserait au moins 1 heure pour l'évaluation globale non invasive de l'état métabolique du greffon par spectroscopie de résonance magnétique (SRM) et imagerie par résonance magnétique (IRM) sans rupture de la chaîne de stérilité dans la mesure où l'étude peut se faire dans le sac de conservation. Or, à ce jour, aucune solution connue ne permet une conservation de l'organe cardiaque pendant une durée supérieure à 4 heures, et a fortiori pendant une durée de 12 heures.

L'un des buts de l'invention est de proposer une solution de qualité améliorée, utilisée pour arrêter le coeur puis pour le conserver pendant une durée supérieure à 4 heures, en vue de la transplantation.

L'un des autres aspects de l'invention est de proposer une solution d'arrêt et de préservation de l'organe, notamment de l'organe cardiaque, permettant de maintenir une intégrité cellulaire et métabolique des organes après ischémie.

Un autre aspect de l'invention est de proposer une solution de conservation d'organes, notamment de l'organe cardiaque, permettant une récupération fonctionnelle post ischémique.

L'invention concerne une solution de perfusion et/ou de conservation et/ou de reperfusion lors de la transplantation d'organes, notamment de l'organe cardiaque, caractérisé en ce qu'elle contient les éléments suivants :

| | |
|---|---|
| K⁺ | à raison d'environ 4 à environ 7 mM, notamment environ 4 mM |
| Ca²⁺ | à raison d'environ 0,2 à environ 0,3 mM, notamment environ 0,25 mM |
| Mg²⁺ | à raison d'environ 13 à environ 16 mM, notamment environ 13 mM |
| glutamate | à raison d'environ 18 à environ 22 mM, notamment environ 20 mM |
| arginine | à raison d'environ 2 à environ 4 mM, notamment environ 3 mM |
| adénosine | à raison d'environ 0,5 à environ 1 mM, notamment environ 0,5 mM |

La solution développée dans le cadre de la présente invention pour l'arrêt et la préservation des coeurs pour une ischémie de longue durée est telle qu'elle induit l'arrêt cardiaque avec une forte concentration en magnésium plutôt qu'en potassium. Elle comprend par ailleurs du glutamate (substrat métabolique de l'ATP en anaérobie) et se caractérise par une concentration non nulle en Ca²⁺ et une concentration en Na⁺ de type extracellulaire. A cette solution ont été ajoutés différents agents protecteurs, (lactobionate, raffinose, glutathion -forme réduite-, allopurinol et adénosine). En ce qui concerne certains composés (adénosine, butanedione-2,3-monoxime), une recherche de la concentration optimale a été réalisée. Des travaux récents ont souligné l'importance du dysfonctionnement endothélial dans les dommages liés à la séquence ischémie-reperfusion. De ce fait, a été incluse la L-arginine, précurseur de NO, dans la solution de préservation.

Par solution de reperfusion lors de la transplantation d'organes, on désigne une solution utilisable pendant la greffe d'un organe, pour faire passer l'organe greffé de l'état d'ischémie à l'état cardioplégique.

Selon un mode de réalisation avantageux, la solution de l'invention ne contient pas de butanedione 2,3-monoxime.

Selon un mode de réalisation avantageux, la solution de l'invention contient au moins un agent imperméant choisi parmi l'acide lactobionique, le mannitol et le raffinose.

Par ordre de préférence, l'agent imperméant est choisi panai : l'acide lactobionique, le mannitol et le raffinose

Selon un autre mode de réalisation avantageux, la solution de l'invention contient au moins un agent piégeur de radicaux libres, notamment choisi parmi le glutathion (forme réduite), l'allopurinol, ou le mannitol.

Selon un autre mode de réalisation avantageux, la solution de l'invention contient les éléments suivants :

| | |
|---|---|
| K⁺ | à raison d'environ 4 à environ 7 mM, notamment environ 4 mM |
| Ca²⁺ | à raison d'environ 0,2 à environ 0,3 mM, notamment environ 0,25 mM |
| Mg²⁺ | à raison d'environ 13 à environ 16 mM, notamment environ 13 mM |
| glutamate | à raison d'environ 18 à environ 22 mM, notamment environ 20 mM |
| arginine | à raison d'environ 2 à environ 4 mM, notamment environ 3 mM |
| adénosine | à raison d'environ 0,5 à environ 1 mM, notamment environ 0,5 mM |

- au moins un agent imperméant,
- au moins un agent piégeur de radicaux libres

| | |
|---|---|
| Osmolarité | 340 mOsm |
| pH | 7,4 |

Selon un autre mode de réalisation avantageux, la solution de l'invention contient les éléments suivants :

| | |
|---|---|
| K⁺ | d'environ 4 à environ 7 mM |
| Ca²⁺ | d'environ 0,2 à environ 0,3 mM |
| Na⁺ | d'environ 108 à environ 132 mM |
| Mg²⁺ | d'environ 13 à environ 16 mM |
| glutamate | d'environ 18 à environ 22 mM |
| arginine | d'environ 2 à environ 4 mM |
| adénosine | d'environ 0,5 à environ 1 mM |
| mannitol | d'environ 27 à environ 33 mM |
| allopurinol | d'environ 0,9 à environ 1,1 mM |
| glutathion (forme réduite) | d'environ 2,7 à environ 3,3 mM |
| raffinose | d'environ 25 à environ 35 mM |
| acide lactobionique | d'environ 80 à environ 120 mM |
| pH | d'environ 7,2 à environ 7,4 |
| osmolarité | d'environ 330 à environ 360 mOsm |

Dans la solution de l'invention, Na⁺ est sous forme NaOH, K⁺ est sous forme KCl et KH₂ PO₄, avantageusement à raison d'environ 2 à environ 3,5 mM KCl et d'environ 2 à environ 3,5 mM KH₂ PO₄, Ca²⁺ est sous forme de Ca Cl₂, 2H₂O et Mg²⁺ est sous forme MgCl₂, 6H₂O.

La solution de l'invention est telle qu'elle permet la conservation d'organes, notamment de l'organe cardiaque pendant une durée d'au moins 12 à 15 heures et notamment pendant au moins 12 heures.

L'invention concerne également une solution telle que définie ci-dessus, pour maintenir l'intégrité cellulaire et métabolique de l'organe après ischémie, cette intégrité pouvant être détectée par mesure des activités de l'un au moins des enzymes suivantes : lactate déshydrogénase, créatine kinase et/ou par mesure de l'un au moins des métabolites suivants : purines, nucléotides notamment nucléotides adényliques, inosine monophosphate, adénosine triphosphate, acides aminés, phosphate inorganique, lactate, phosphocréatine.

L'invention concerne également une solution telle que définie ci-dessus, pour assurer la récupération fonctionnelle post ischémique, cette récupération fonctionnelle pouvant être mesurée par la mesure d'un au moins des paramètres hémodynamiques suivants : le débit coronaire, la pression développée, la fréquence cardiaque, et la pression diastolique.

La qualité de la préservation est mesurée par l'étude de paramètres fonctionnels et métaboliques ainsi que par la mesure de l'intégrité cellulaire.

Les paramètres fonctionnels mesurés sont :
- la pression développée,
- la pression diastolique,
- la fréquence cardiaque, ces trois paramètres étant mesurés grâce à un ballonnet placé dans le ventricule gauche sur lequel se contracte le coeur ; le ballonnet est relié à un amplificateur (Statham P23dB) qui est connecté à un enregistreur,
- et le débit coronaire(mesure des effluats sortant du coeur pendant un temps donné).

Les paramètres fonctionnels sont mesurés pendant la période de contrôle et pendant la période de reperfusion.

Les paramètres métaboliques mesurés sont :
- le phosphate inorganique,
- l'adénosine triphosphate,
- la phosphocréatine, et
- le pH intracellulaire.

L'acquisition de spectres de RMN du P-31 permet de suivre les niveaux de Pi (phosphate inorganique), ATP (adénosine triphosphate), PCr (phosphocréatine) ainsi que le pH intracellulaire (ATP et PCr = composés phosphorés à haute énergie). Les spectres sont acquis pendant toute la durée de l'expérience (contrôle, ischémie, reperfusion).

Les nucléotides adényliques, l'inosine monophosphate et les purines sont mesurés dans les coeurs congelés en fin d'expérience ; cette mesure est complémentaire des résultats obtenus à partir des spectres de RMN du P-31.

Les acides aminés sont mesurés dans les coeurs congelés à la fin des expériences. Ils jouent un rôle important dans le métabolisme et le maintien de la fonction cellulaire cardiaque. Ils sont déplétés pendant l'ischémie et la reperfusion. L'amplitude de cette déplétion est un reflet de l'altération du coeur.

Le phosphate inorganique (Pi) et les purines sont mesurés dans les effluats sortant du coeur et donc sont mesurés uniquement pendant les périodes de perfusion (contrôle et reperfusion). Le Pi et les purines sont accumulés durant l'ischémie et sortent du coeur au moment de la reperfusion, représentant une perte de précurseurs pour la synthèse des composés à haute énergie.

Le lactate dans les effluats (contrôle et reperfusion) est évalué en tant qu'indice du métabolisme énergétique en anaérobie.

Paramètres reflétant l'intégrité cellulaire.

La fuite de créatine kinase (CK) dans les effluats indique un dommage cellulaire.

Les quantités de créatine kinase (CK) et de lactate déshydrogénase (LDH) dans les coeurs congelés à la fin de l'expérience représentent également un indice de l'intégrité cellulaire.

Le contenu myocardique en eau reflète également l'intégrité cellulaire.

Afin de mettre en évidence l'intérêt présenté par la solution de l'invention, celle-ci a été comparée, à l'aide d'un grand nombre de paramètres et vis-à-vis de la protection apportée, aux solutions de référence que sont la solution de St Thomas et la solution UW, utilisées en matière de préservation des greffons cardiaques. Une comparaison avec les solutions Broussais et Celsior, utilisées dans certains centres de transplantation français a été également faite. Les résultats montrent que la solution de l'invention permet une protection supérieure à celle apportée par les autres solutions : St Thomas, UW, Broussais, Celsior (cf. figures).

La solution de l'invention peut être utilisée pendant toutes les phases d'une transplantation :
1) pour arrêter le coeur du donneur (solution cardioplégique),
2) pour conserver le coeur (stockage hypothermique, transport),
3) pendant la réimplantation chez le receveur.

Les solutions de l'invention peuvent être préparées de la façon suivante : les différents constituants sont dissous et dilués dans de l'eau distillée, le pH est ajusté à 7,4 avec NaOH, La solution est filtrée sur 0,2 µm. La solution apyrogène et stérile est préparée et conservée à l'abri de l'oxygène de l'air.

### ABREVIATIONS UTILISEES (figures, texte)

- ATP =: adénosine triphosphate
- ADP =: adénosine diphosphate
- AMP =: adénosine monophosphate
- PCr =: phosphocréatine
- Pi =: phosphate inorganique
- LDH =: lactate déshydrogénase
- CK =: créatine kinase
- HPLC ou CLHP =: chromatographie liquide haute pression
- SRM =: spectroscopie de résonance magnétique
- EDP =: pression diastolique
- dP =: pression développée
- CF =: débit coronaire
- RPP =: "rate pressure product" = fréquence cardiaque x pression développée
- NAD =: nicotinamide adénine dinucléotide.

Dans ce qui suit, une solution conforme à l'invention, utilisable pour des coeurs isolés, présente la composition suivante :

KCl (2 mM), KH₂PO₄ (2 mM), CaCl₂ (0,25 mM), MgCl₂ (13 mM), NaOH (120 mM), arginine (2 mM), glutamate (20 mM), adénosine (0,5 mM), acide lactobionique (100 mM), raffinose (30 mM), glutathion (3 mM), allopurinol (1 mM), mannitol (30 mM).

Une solution conforme à l'invention, utilisable pour une transplantation hétérotopique, présente la composition suivante :

KCl (2 mM), KH₂PO₄ (2 mM), CaCl₂ (0,25 mM), MgCl₂ (13 mM), NaOH (120 mM), arginine (3 mM), glutamate (20 mM), adénosine (0,5 mM), acide lactobionique (100 mM), raffinose (30 mM), glutathion (3 mM), allopurinol (1 mM), mannitol (30 mM).

Une solution de composition proche de celle de l'invention, ne contenant pas d'arginine, est dénommée dans ce qui suit « CRMBM » et présente la composition suivante :

KCI (2 mM), KH₂PO₄ (2 mM), CaCl₂ (0,25 mM), MgCl₂ (13 mM), NaOH (120 mM), glutamate (20 mM), adénosine (0,5 mM), acide lactobionique (100 mM), raffinose (30 mM), glutathion (3 mM), allopurinol (1 mM), mannitol (30 mM). **(J. Heart Lung. Transplant 1999; 18: 572-581).**

### DESCRIPTION DES FIGURES

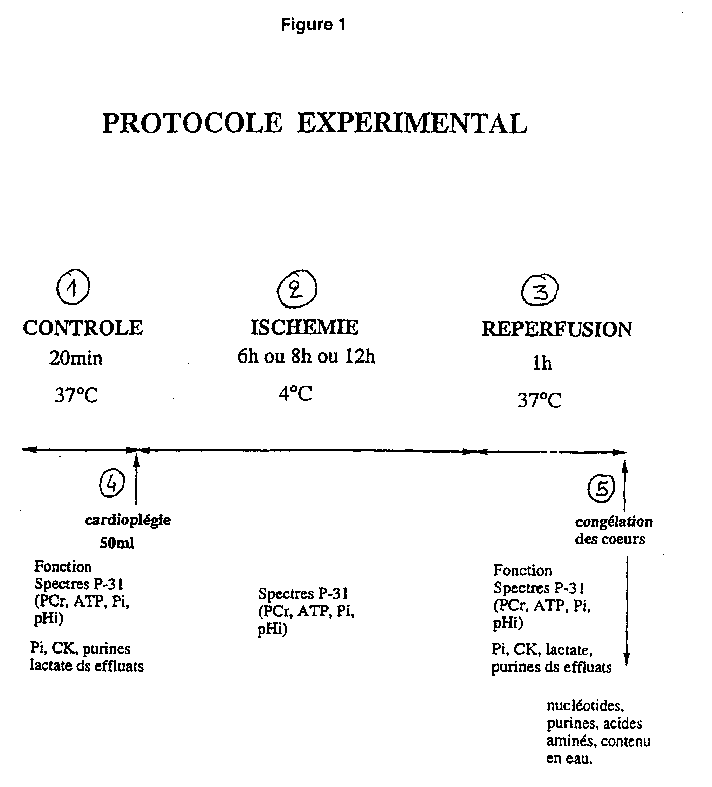

La figure 1 représente le protocole expérimental utilisé dans les exemples 1 et 2 décrits ci-après.

Sur la figure 1, ① ② ③ ④ et ⑤ ont les significations suivantes :
① correspond au contrôle (20 mn, 37°C) ;
   on mesure PCr, ATP, Pi, pHi par spectres P-31, et on mesure Pi, CK, purines et lactate dans les effluats.
② correspond à l'ischémie (6h ou 8h ou 12h à 4°C)
   on mesure PCr, ATP, Pi, pHi par spectres P-31.
③ correspond à la reperfusion pendant 1h à 37°C ;
   on mesure :
   - PCr, ATP, Pi, pHi par spectres P-31
   - Pi, CK, lactate et purines dans les effluats pendant la reperfusion ;
   on mesure ensuite les nucléotides, purines, acides aminés et le contenu en eau après la congélation des coeurs.
④ correspond à l'étape de cardioplégie (50 ml).
⑤ correspond à la congélation des coeurs.

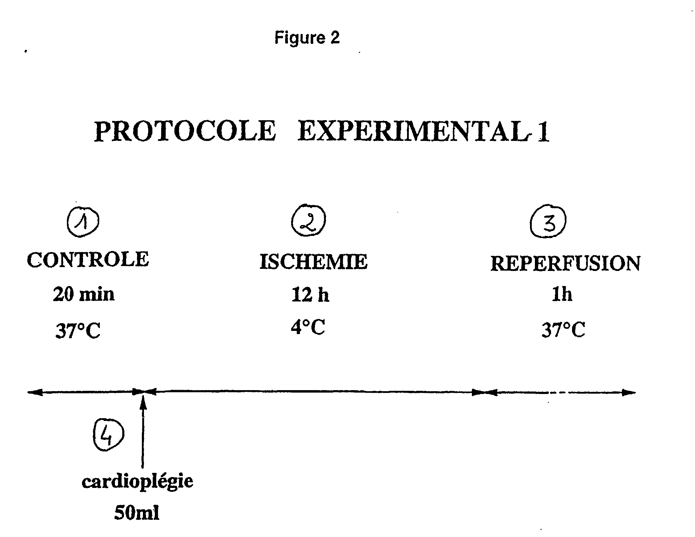

La figure 2 représente le protocole expérimental n° 1 (voir exemple comparatif 3).

Sur la figure 2, ① ② ③ et ④ ont les significations suivantes :
① contrôle (20 mn, 37°C).
② ischémie (12h, 4°C).
③ reperfusion (1h, 37°C).
④ correspond à l'étape de cardioplégie (50 ml).

Les figures 3A et 3B représentent, dans le cadre du protocole n° 1, la cinétique des paramètres hémodynamiques au cours de la reperfusion à l'aide respectivement de la solution de St Thomas (repérée par des carrés noirs), de la solution de Broussais (repérée par des carrés blancs), de la solution UW (repérée par des ronds noirs), de la solution CRMBM (repérée par des ronds blancs).

*p<0.0001 vis-à-vis de la solution de Broussais, p< 0.001 vis-à-vis des solutions UW et St Thomas

†p<0.0001 vis-à-vis de la solution de Broussais, et UW, p< 0.01 vis-à-vis de la solution St Thomas.

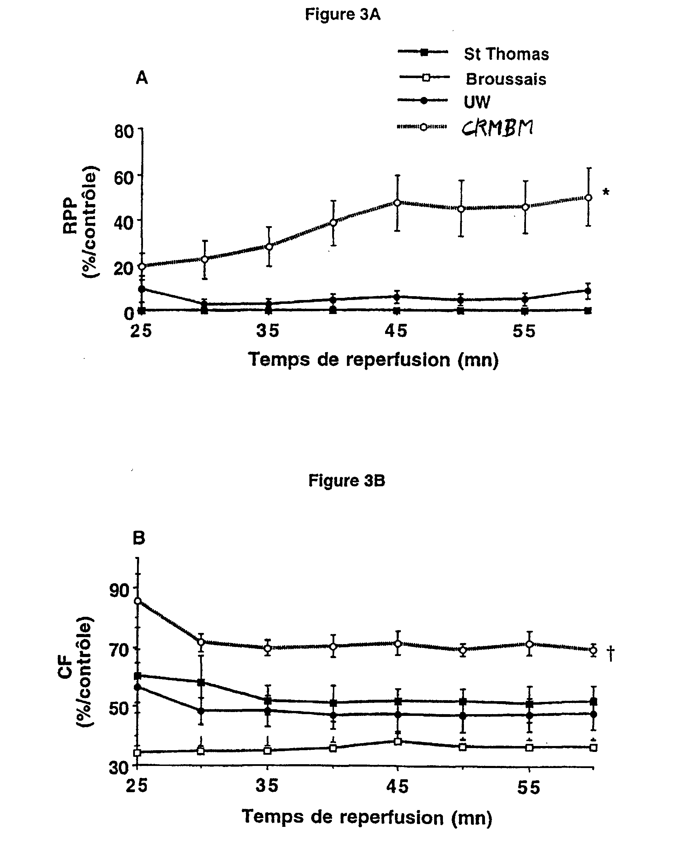

La figure 3A représente le produit de la fréquence cardiaque par la pression développée (RPP) (pourcentage par rapport au contrôle) exprimé en fonction du temps de reperfusion (en minutes).

La figure 3B représente le débit coronaire (CF) (pourcentage par rapport au contrôle) exprimé en fonction du temps de reperfusion en minutes.

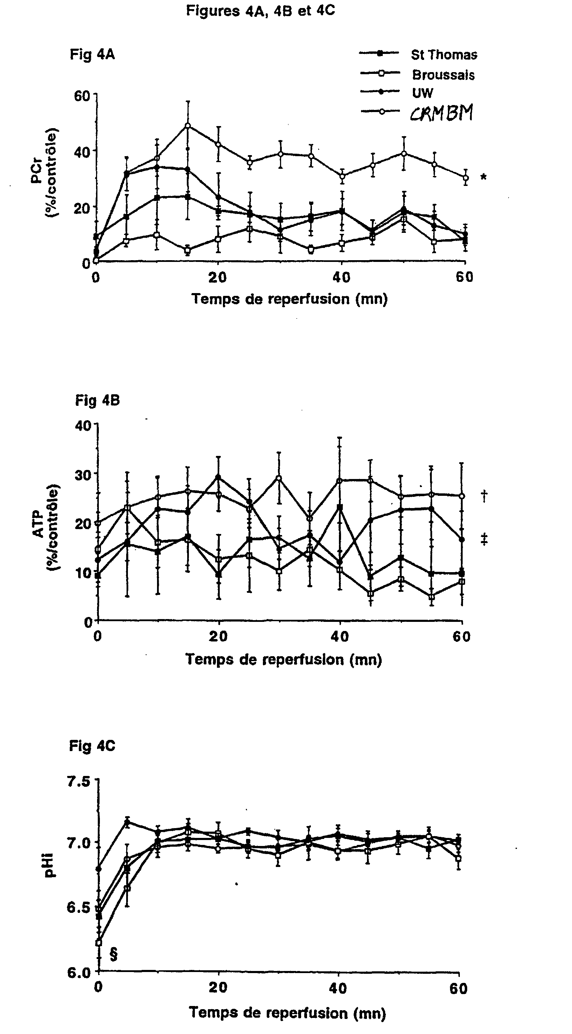

La figure 4A représente, dans le cadre du protocole n° 1, la variation de la phosphocréatine (PCr) (pourcentage par rapport au contrôle) en fonction du temps de reperfusion (exprimé en minutes).

La figure 4B représente, dans le cadre du protocole n° 1, la variation de l'adénosine triphosphate (ATP) (pourcentage par rapport au contrôle) en fonction du temps de reperfusion (exprimé en minutes).

La figure 4C représente, dans le cadre du protocole n° 1, la variation de pH intracellulaire (pHi).

Dans les figures 4A, 4B et 4C, le temps 0 correspond à la fin de l'ischémie.

Dans les figures 4A, 4B, 4C, on a représenté respectivement la solution de St Thomas (repérée par des carrés noirs), la solution de Broussais (repérée par des carrés blancs), la solution UW (repérée par des ronds noirs), la solution CRMBM (repérée par des ronds blancs).

Dans les figures 4A, 4B, 4C
*p<0.05 vis-à-vis de la solution UW, p < 0.001 vis-à-vis des solutions Broussais et St Thomas,
†p<0.001 vis-à-vis de la solution Broussais, p<0.01 vis-à-vis de la solution St Thomas,
‡p < 0.0001 vis-à-vis de la solution Broussais. p<0.05 vis-à-vis de la solution UW.

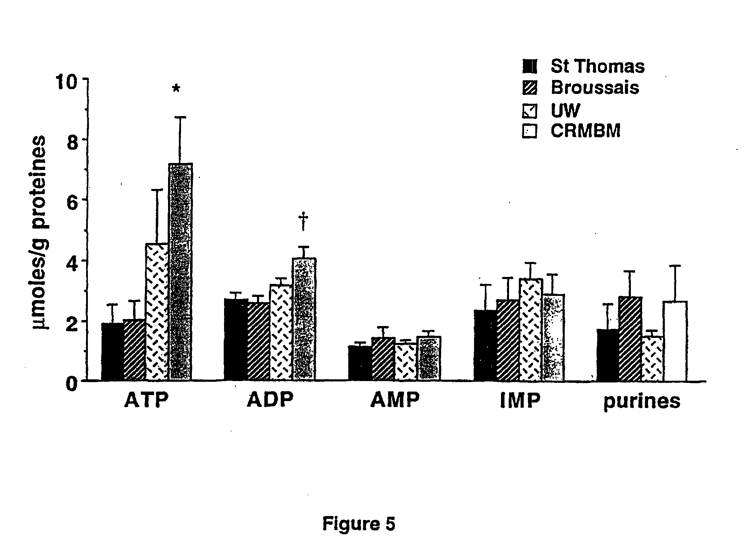

La figure 5 représente, dans le cadre du protocole n° 1, les contenus en nucléotides adényliques (ATP = adénosine triphosphate, ADP = adénosine diphosphate, AMP = adénosine monophosphate, IMP (inosine monophosphate), et purines, dans les coeurs congelés à la fin de l'ischémie). Ces contenus sont exprimés en micromoles par gramme de protéines.

La solution de St Thomas est représentée en noir, la solution de Broussais est représentée en hachure noir et blanc. La solution UW est représentée par des pointillés perpendiculaires et la solution CRMBM, de composition proche de celle de l'invention, est représentée en gris.

Sur la figure 5
*p < 0.05 vis-à-vis de la solution Broussais et St Thomas,
†p < 0.05 vis-à-vis de la solution de Broussais.

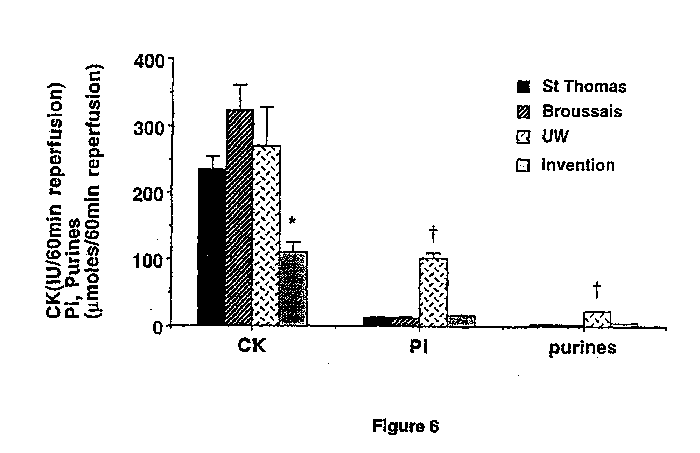

La figure 6 représente, dans le cadre du protocole n° 2, les métabolites dans les effluats coronaires au cours de la reperfusion. Ces métabolites sont :
- CK (créatine kinase) exprimée en unités internationales par 60 mn de reperfusion,
- Pi (phosphate inorganique) exprimé en micromoles par 60 minutes de reperfusion,
- purines exprimées en micromoles par 60 minutes de reperfusion.

Sur la figure 6
*p<0.001 vis-à-vis des solutions St Thomas et UW, p<0.001 vis-à-vis de la solution de Broussais,
†p < 0.0001 vis-à-vis de la solution de l'invention, Broussais et St Thomas.

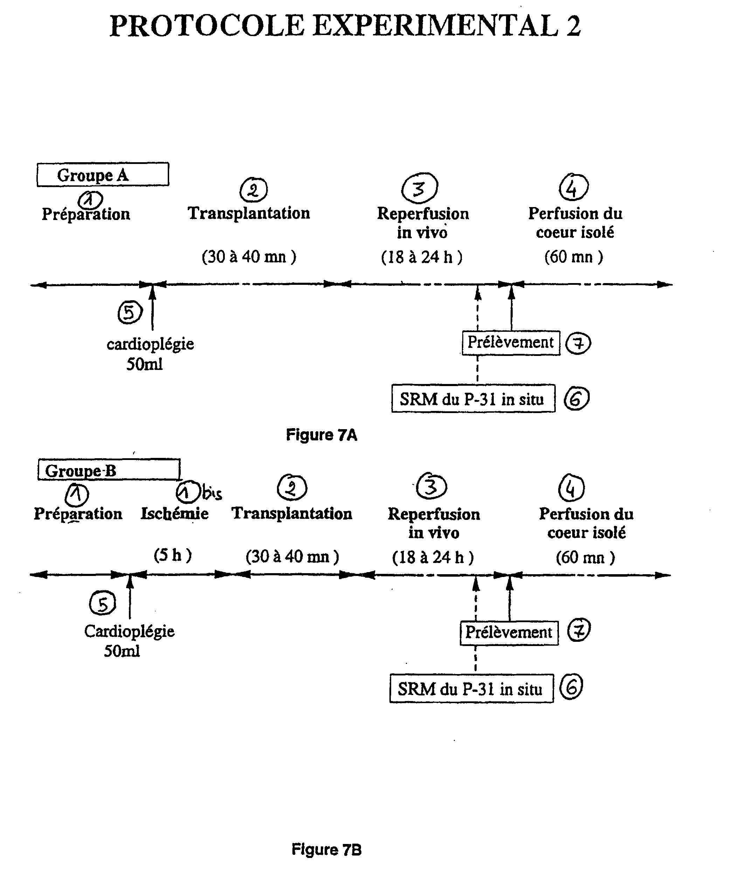

Les figures 7A et 7B correspondent au protocole expérimental n° 2.

La figure 7A correspond au groupe A (défini dans le protocole n° 2).

La figure 7 B correspond au groupe B (défini dans le protocole n° 2).

Sur les figures 7A et 7B, ① ② ③ ④ ⑤ ⑥ ⑦ et ① **bis** ont les significations suivantes :

| | | |
|---|---|---|
| ① | préparation | ①**bis** ischémie (5h). |
| ② | transplantation (30 à 40 mn). | |
| ③ | reperfusion *in vivo* (18 à 24h). | |
| ④ | perfusion du coeur isolé (60 mn). | |
| ⑤ | cardioplégie (50 ml). | |
| ⑥ | SRM du P-31 *in situ.* | |
| ⑦ | prélèvement du coeur. | |

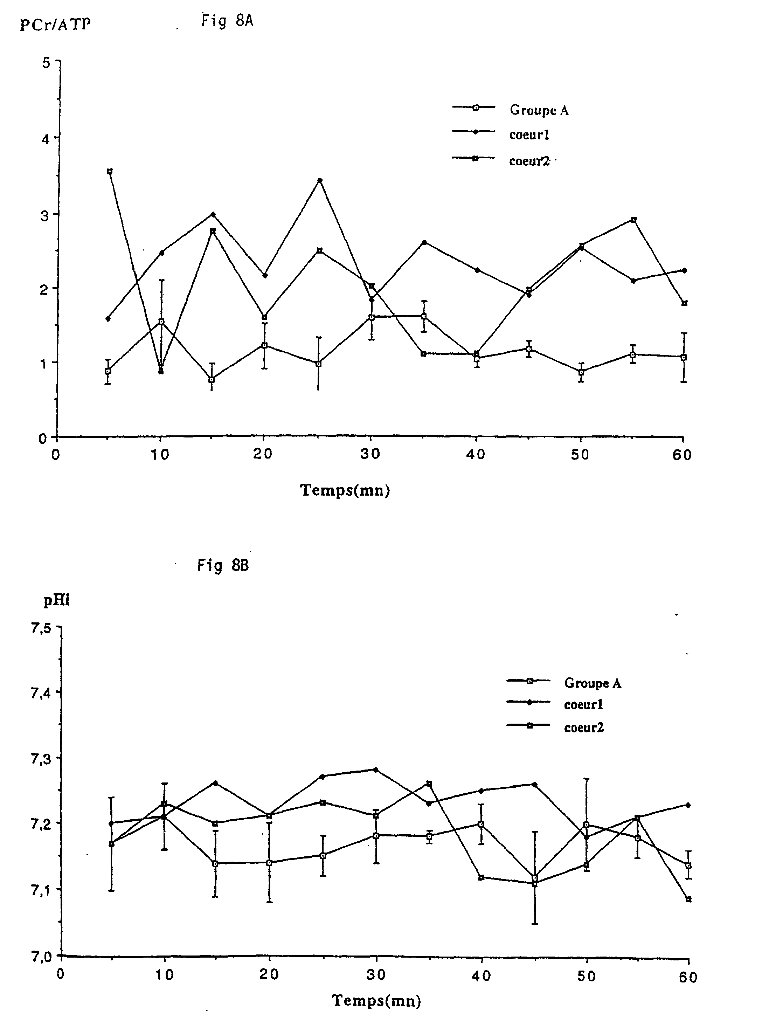

La figure 8A correspond, dans le cadre du protocole n° 2, à la variation du rapport PCr/ATP exprimé en fonction du temps (en minutes).

La figure 8 B correspond à la variation de pH intracellulaire (pHi) exprimé en fonction du temps (en minutes).

Le groupe B correspond à l'ensemble coeur 1 et coeur 2.

Les résultats du groupe A correspondent à la moyenne de 4 expériences.

Les résultats du groupe B sont représentés par 2 expériences (coeur 1 et coeur 2).

Les courbes avec des carrés blancs comportant un point en leur centre, correspondent au groupe A, les courbes avec des losanges noirs correspondent au coeur 1 (groupe B) et les courbes comportant des carrés blancs correspondent au coeur 2 (groupe B).

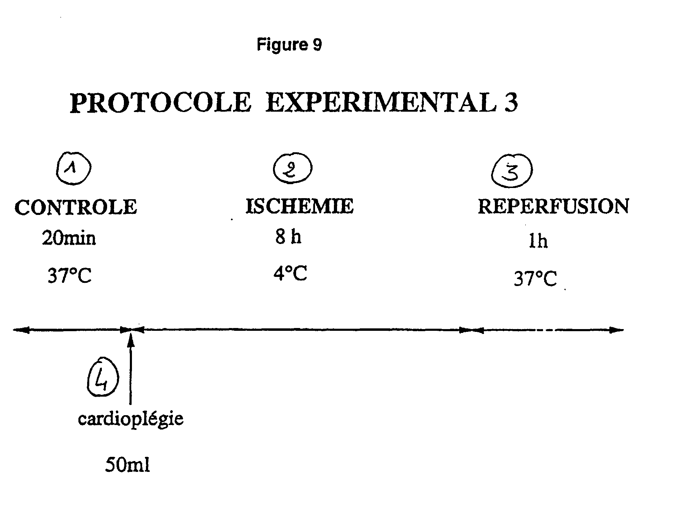

La figure 9 représente le protocole expérimental n° 3.

Sur la figure 9, ① ② ③ et ④ ont les significations suivantes :
① contrôle (20 mn, 37°C).
② ischémie (8h, 4°C).
③ reperfusion (1h, 37°C).
④ cardioplégie (50 ml).

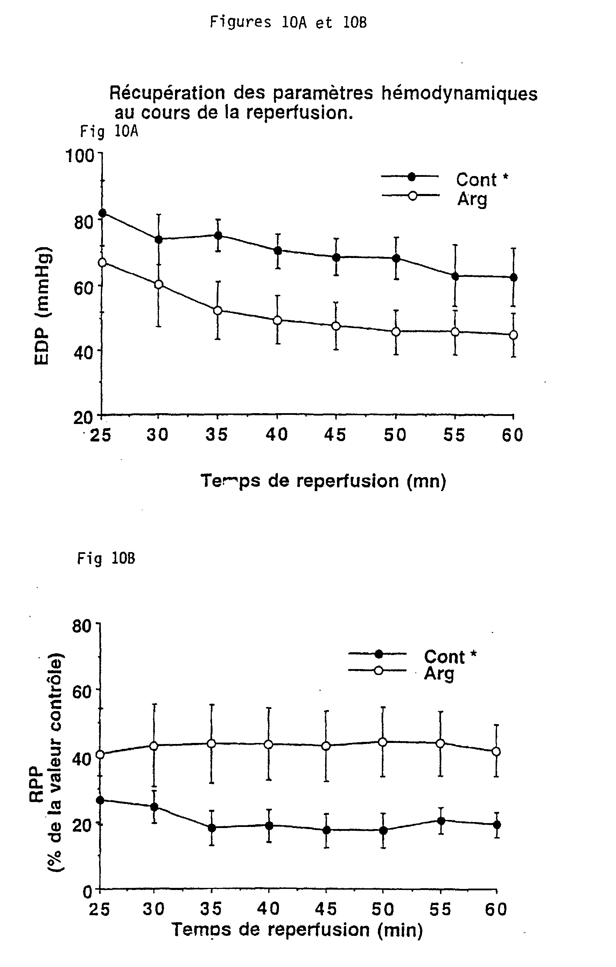

La figure 10A correspond, dans le cadre du protocole n° 3, à la variation de pression diastolique (EDP) (en mm de Hg), exprimée en fonction du temps de reperfusion (en minutes).

La figure 10B correspond, dans le cadre du protocole n° 3, à la variation du produit de la pression développée par la fréquence cardiaque (RPP) (exprimé en pourcentage de la valeur contrôle) en fonction du temps de reperfusion (en minutes).

Dans les figures 10 A et 10B les courbes comportant des ronds blancs correspondent à la présence d'arginine et les courbes comportant des ronds noirs correspondent à la solution de contrôle.

p < 0,0001 dans le cadre de la courbe correspondant à la solution de contrôle contenant de l'arginine.

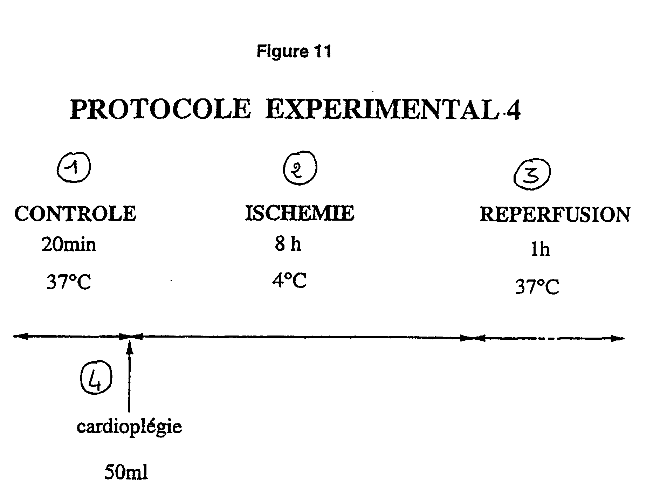

La figure 11 correspond au protocole expérimental n° 4.

Sur la figure 11, ① ② ③ et ④ ont les significations suivantes :
① contrôle (20 mn, 37°C).
② ischémie (8h, 4°C).
③ reperfusion (1h, 37°C).
④ cardioplégie (50 ml).

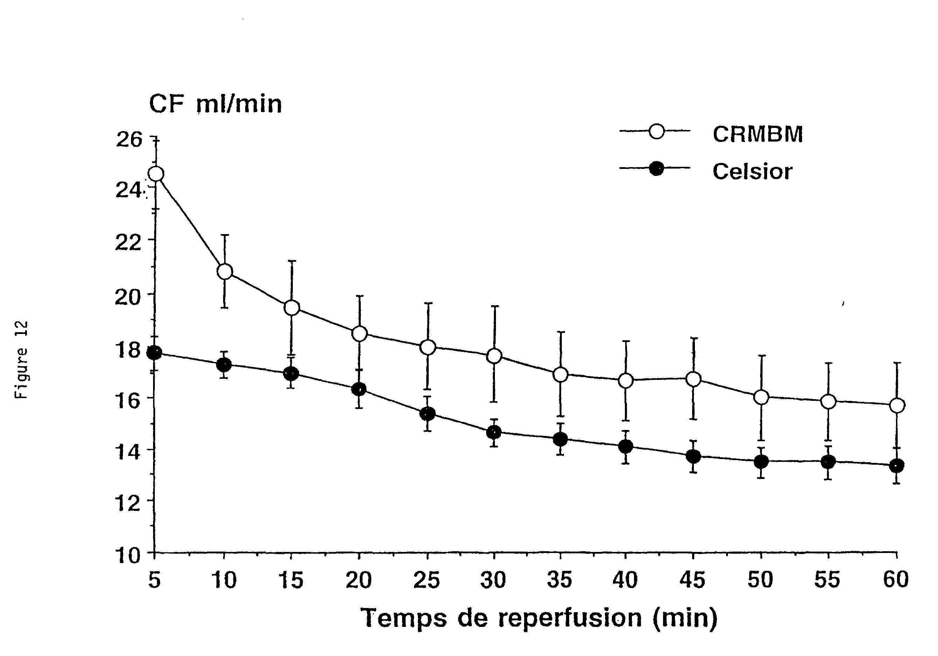

La figure 12 correspond, dans le cadre du protocole n° 4, à la variation du débit coronaire (CF) (exprimé en ml/mn) en fonction du temps de reperfusion (en minutes),

La courbe avec des ronds noirs correspond à la solution Celsior et la courbe avec des ronds blancs correspond à la solution de l'invention.

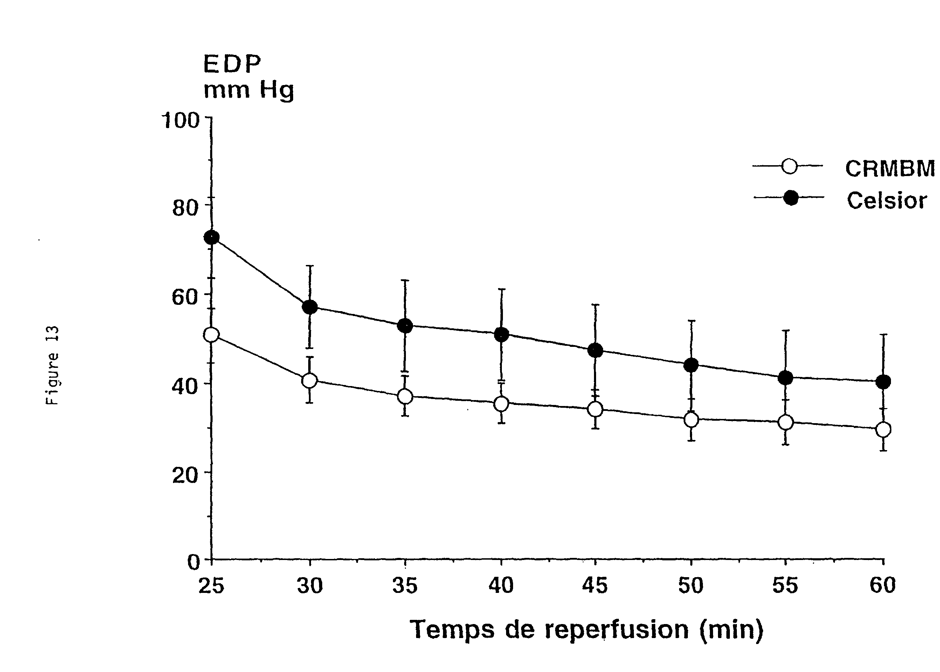

La figure 13 correspond, dans le cadre du protocole n° 4, à la variation de pression diastolique (EDP) (exprimé en mm/Hg) en fonction du temps de reperfusion (en minutes).

La courbe avec des ronds noirs correspond à la solution Celsior et la courbe avec des ronds blancs correspond à la solution de l'invention.

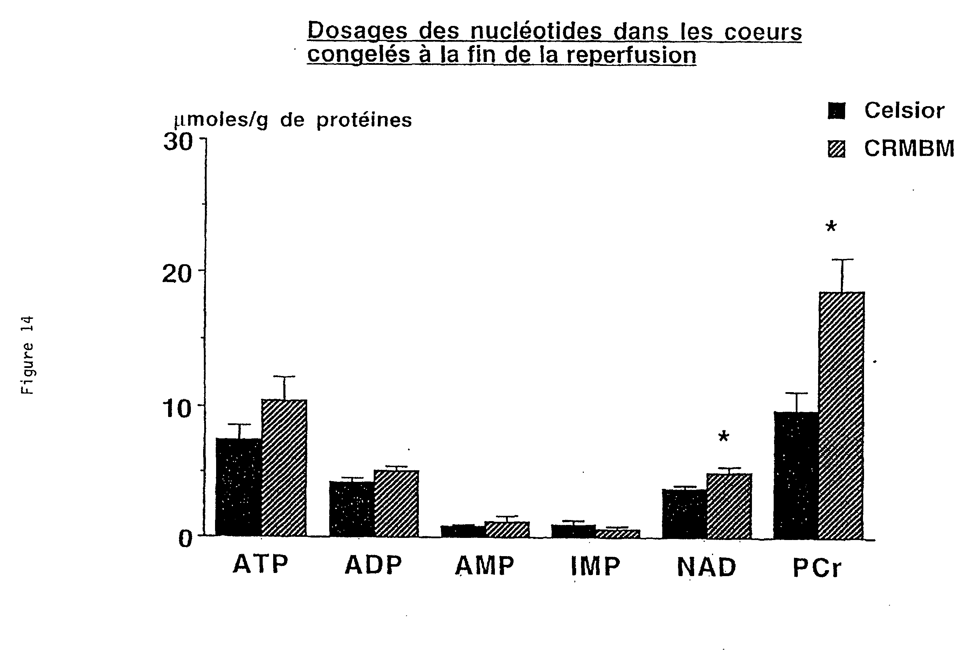

La figure 14 correspond, dans le cadre du protocole n° 4, au dosage des nucléotides dans les coeurs congelés à la fin de la reperfusion. On a exprimé ainsi le dosage d'ATP (adénosine triphosphate), d'ADP (adénosine diphosphate), d'AMP (adénosine monophosphate), d'IMP (inosine monophosphate), de NAD (nicotinamide adénine dinucléotide), de PCr (phosphocréatine), en micromoles par grammes de protéines.

La solution Celsior est représentée en noir et la solution de l'invention est représenté par des hachures.

p < 0,05 dans le cadre de la solution de l'invention vis-à-vis de la solution de Celsior.

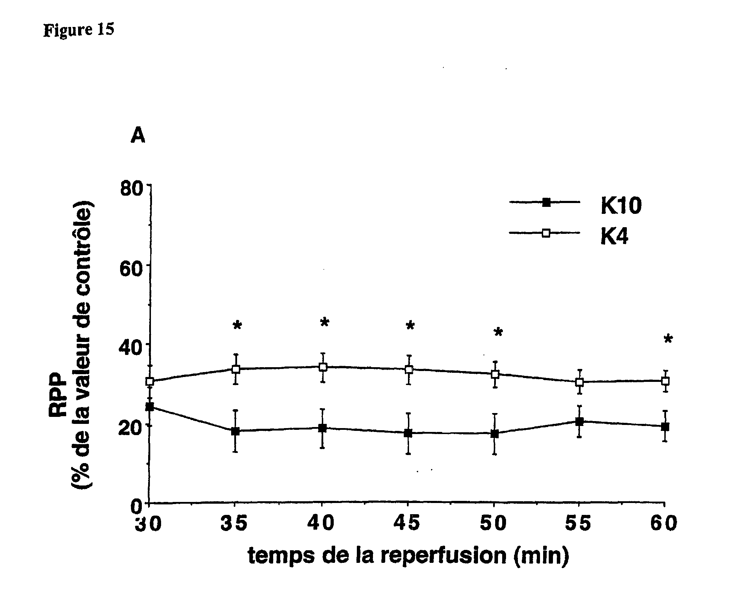

La figure 15 représente, dans le cadre du protocole expérimental utilisé dans l'exemple 3 ci-après, le pourcentage de récupération de la fonction myocardique, exprimée sous forme du produit de la pression développée par la fréquence cardiaque (RPP), en fonction du temps de la reperfusion (en minutes) en présence de 4 mM ou 10 mM de potassium.

La solution de préservation contenant 4 mM de potassium est repérée par des carrés blancs, et celle contenant 10 mM de potassium est repérée par des carrés noirs.

Dans la figure 15, *p < 0,05 vis-à-vis de la solution contenant 10 mM de potassium.

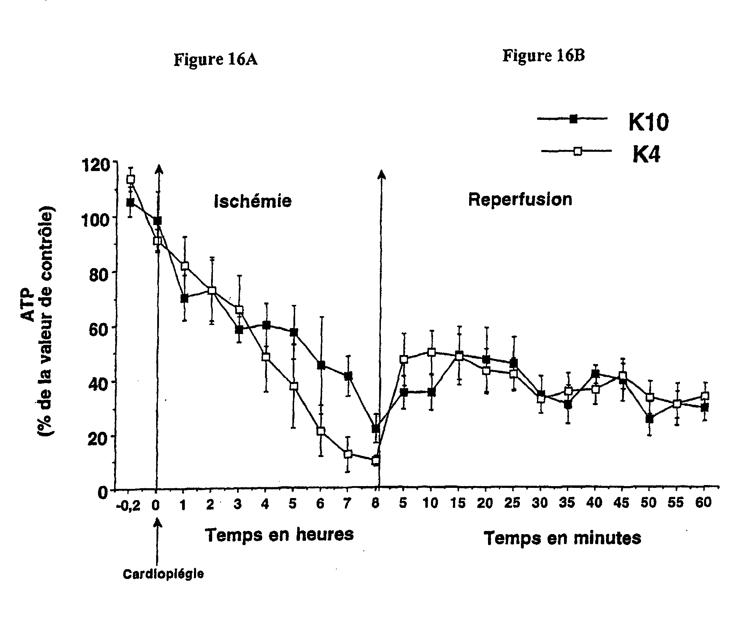

Les figures 16A et 16B représentent respectivement, dans le cadre du protocole expérimental utilisé dans l'exemple 3, la variation de l'ATP en fonction (1) du temps d'ischémie (exprimé en heures) (figure 16A), et (2) du temps de reperfusion (exprimé en minutes) (figure 16B), en présence de 4 mM ou de 10 mM de potassium.

Dans la figure 16A, le temps 0 correspond au départ de l'ischémie, et les valeurs négatives correspondent à l'enregistrement pré-ischémique (perfusion contrôle).

Dans la figure 16B, le temps 0 correspond à la fin de l'ischémie et au départ de la reperfusion.

Dans les figures 16A et 16B, la solution de préservation contenant 4 mM de potassium est repérée par des carrés blancs, et celle contenant 10 mM de potassium est repérée par des carrés noirs.

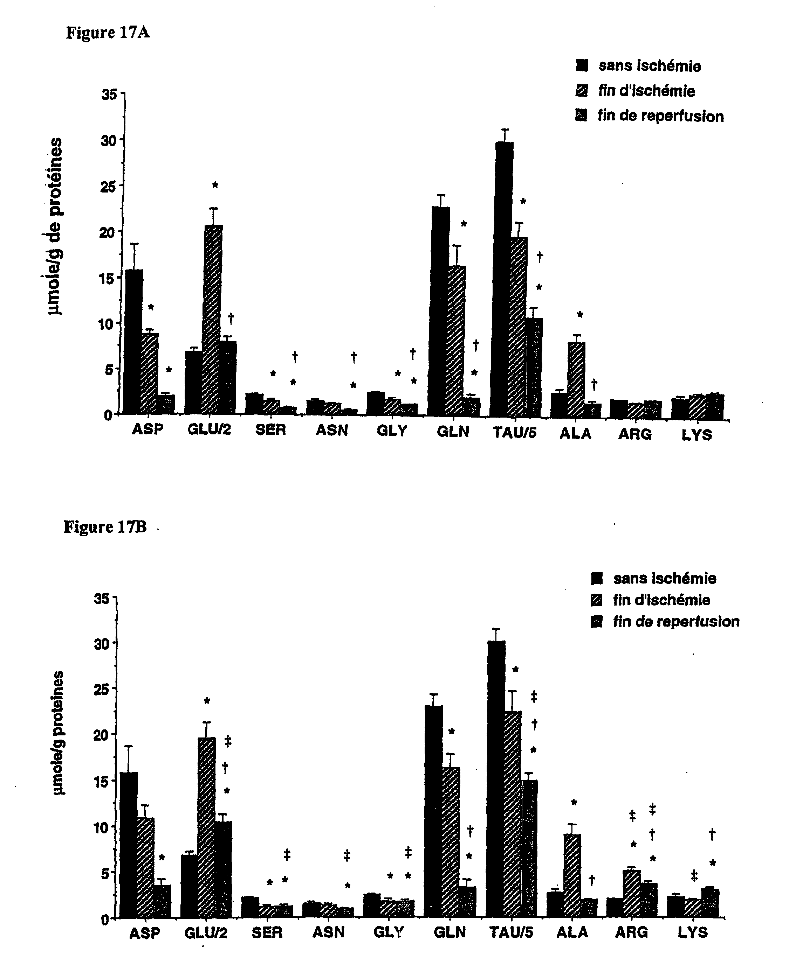

Les figures 17A et 17B représentent respectivement, dans le cadre du protocole expérimental 3 (correspondant à 8 H d'ischémie et 60 minutes de reperfusion), les contenus en acides aminés (ASP = Acide aspartique, GLU = Acide glutamique, SER = Sérine, ASN = Asparagine, GLY = Glycine, GLN = Glutamine, TAU = protéine Taurine, ALA = Alanine, ARG = Arginine, LYS = Lysine) dans les coeurs sans ischémie, en fin d'ischémie, et en fin de reperfusion (1) pour les coeurs sans arginine (Figure 17A), et (2) pour les coeurs avec arginine (Figure 17B).

L'abréviation « GLU/2 » représente la concentration de glutamine divisée par 2, et « TAU/5 » la concentration de taurine divisée par 5 (ceci pour des problèmes d'échelle, afin de pouvoir mettre tous les acides aminés sur le même graphe).

Les contenus sont exprimés en micromoles par gramme de protéines.

Dans les figures 17A et 17B, l'histogramme noir représente les contenus en acides aminés dans les coeurs sans ischémie, l'histogramme hachuré en noir et blanc les contenus en acides aminés dans les coeurs en fin d'ischémie, l'histogramme gris les contenus en acides aminés dans les coeurs en fin de reperfusion.

Dans les figures 17A et 17B :
*p<0.05 vis-à-vis des coeurs sans ischémie,
†p<0.05 vis-à-vis des coeurs en fin d'ischémie.

Dans la Figure 17B :
p < 0.05 vis-à-vis du groupe contrôle.

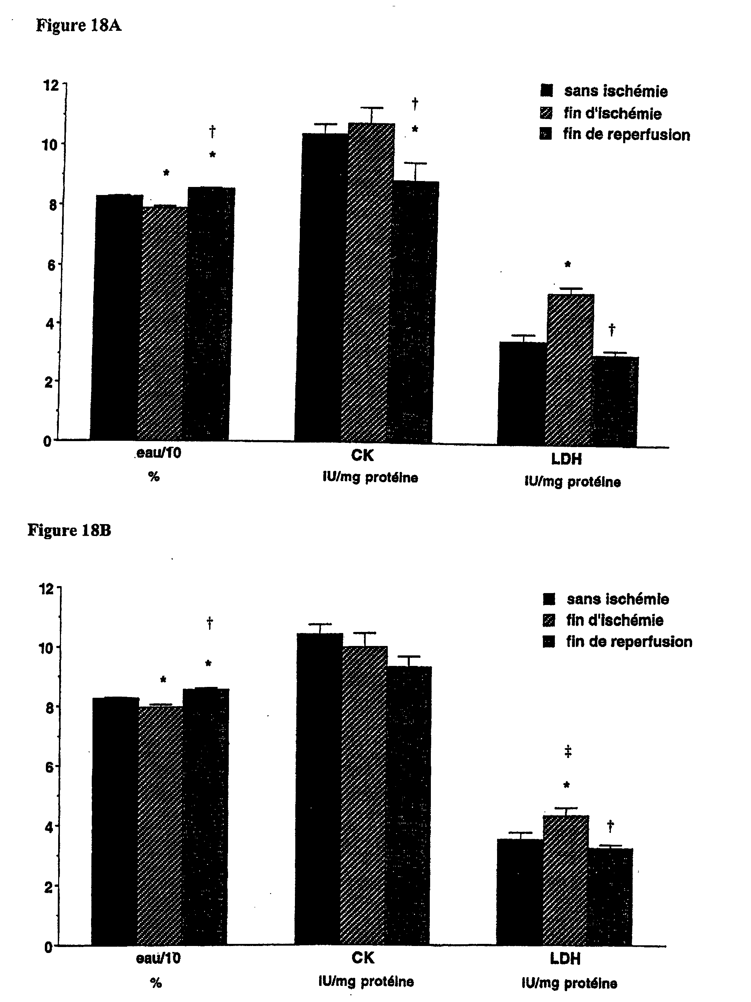

Les figures 18A et 18B représentent respectivement de gauche à droite, dans le cadre du protocole expérimental 3, les contenus en eau (exprimé en %), en créatine kinase (CK) et en lactate déshydrogénase (LDH) (exprimés en unités internationales par mg de protéine) dans les coeurs sans ischémie, en fin d'ischémie et en fin de reperfusion (1) pour les solutions ne contenant pas d'arginine (figure 18A) et (2) pour les solutions selon l'invention contenant de l'arginine (figure 18B).

Dans les figures 18A et 18B, l'histogramme noir représente les contenus en eau, CK et LDH dans les coeurs sans ischémie, l'histogramme hachuré en noir et blanc les contenus en eau, CK et LDH dans les coeurs en fin d'ischémie, l'histogramme gris les contenus en eau, CK et LDH dans les coeurs en fin de reperfusion.

Dans les figures 18A et 18B :
*p<0.05 vis-à-vis des coeurs sans ischémie,
†p < 0.05 vis-à-vis des coeurs en fin d'ischémie.

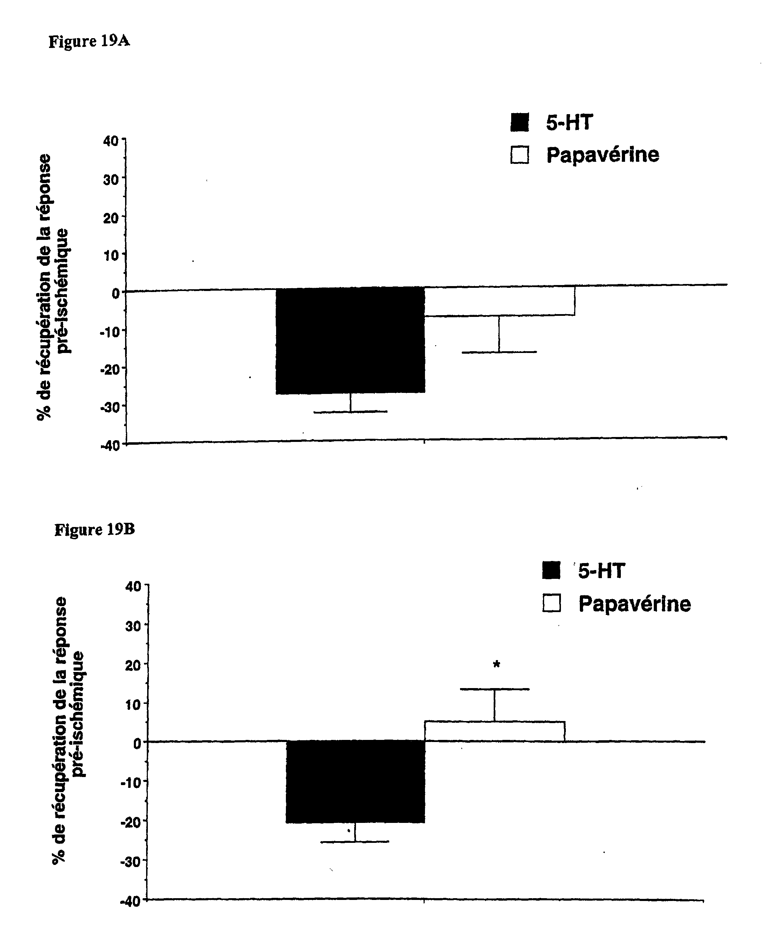

Les figures 19A et 19B représentent respectivement, dans le cadre du protocole 3, le pourcentage de récupération de la réponse pré-ischémique au 5-HT et à la papaverine après 8 heures d'ischémie froide (1) après préservation sans arginine (figure 19A), (2) avec préservation en présence d'arginine (figure 19B).

La réponse au 5-HT est repérée par les carrés noirs, et celle à la papaverine par les carrés blancs.

Dans la figure 19B :
*p<0.05 réponse à la papavérine en comparaison à la réponse au 5-HT

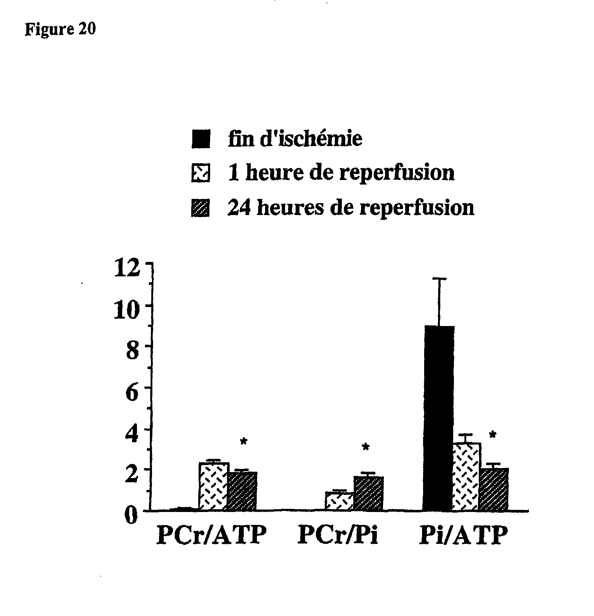

La figure 20 représente, dans le cadre d'un protocole correspondant à 3 heures d'ischémie avec arrêt et conservation dans la solution CRMBM, les rapports de différents métabolites énergétiques après 1 heure de reperfusion (représentés par l'histogramme blanc tâché de traits noirs) et 24 heures de reperfusion (représentés par l'histogramme hachuré en noir et blanc).

L'histogramme noir représente la fin de l'ischémie.
*p<0.05 24 heures de reperfusion en comparaison à 1 heure de reperfusion

Le pool intracellulaire de PCr est totalement déplété à la fin de l'ischémie et est progressivement restauré durant la reperfusion. La figure 20 montre que les rapports PCr/ATP et Pi/ATP diminuent significativement, tandis que le rapport PCr/Pi augmente significativement entre 1 heure et 24 heures de reperfusion.

La fonction cardiaque augmente significativement entre 1 heure et 24 heures de reperfusion, atteignant 1331 ± 81 mmHg/sec et 2081 ± 333 mmHg/sec respectivement.

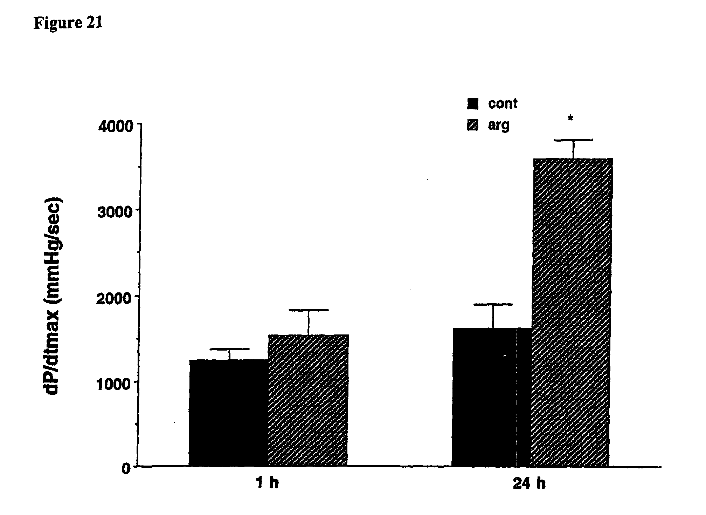

La figure 21 représente, dans le cadre du protocole expérimental correspondant à 3 heures d'ischémie avec arrêt et conservation dans la solution CRMBM (ne contenant pas d'arginine) et dans la solution de l'invention (contenant de l'arginine), (1) la fonction cardiaque en absence ou en présence d'arginine après 1 heure de reperfusion (voir gauche de la figure), (2) la fonction cardiaque en absence ou en présence d'arginine après 24 heures de reperfusion (voir droite de la figure).

La fonction cardiaque, telle que représentée par la dérivée de la pression développée (dP/dtₘₐₓ), est repérée par l'histogramme noir en absence d'arginine, et par l'histogramme hachuré en noir et blanc en présence d'arginine.
*p < 0.05 en présence d'arginine en comparaison à l'absence d'arginine

### EXEMPLES

### EXEMPLE 1 : MODELE DE COEUR ISOLE PERFUSE

### 1.1. Préparation du coeur perfusé

Les coeurs sont prélevés sur des rats mâles Sprague Dawley de 350 à 375g et anesthésiés par injection intrapéritonéale de pentobarbital. Les coeurs sont ensuite perfusés suivant le modèle du coeur contractant isovolumique. Dans ce modèle, le coeur est perfusé avec du tampon Krebs-Henseleit à 37°C, sans phosphate, de façon rétrograde par l'aorte, à une pression constante de 100mmHg. Un ballon incompressible, placé dans le ventricule gauche rend la contraction isovolumique. Ce modèle permet d'évaluer la fonction cardiaque, par la pression développée et de mesurer la compliance par la mesure de la pression diastolique.

### 1.2. Appareillage de perfusion

Le système de perfusion est composé de différents réservoirs thermostatés à 37°C grâce à une circulation continue d'eau. Un circuit indépendant est prévu pour le réservoir contenant la solution de cardioplégie, maintenu à 4 ou 7,5°C. Dans le tube de spectroscopie de résonance magnétique (SRM), les effluats coronaires sont évacués par aspiration grâce à une pompe rotative et sont recueillis à des temps donnés pour la réalisation de différents dosages biochimiques.

### 1.3. Solutions de perfusion

Pendant les périodes de perfusion normoxique, la solution employée est du tampon bicarbonate de Krebs-Henseleit. La solution de cardioplégie utilisée pour arrêter et conserver le coeur est variable.

### 1.4. Expériences de SRM du P-31

Les spectres de SRM du P-31 sont obtenus à 81MHz en utilisant un spectromètre Bruker-Nicolet WP-200 interfacé à un aimant vertical de 4,7 Tesla, équipé d'une sonde sélective de 20mm de diamètre. L'homogénéité du champ magnétique est réalisée en utilisant le signal des protons de l'échantillon contenus dans le volume sensible de la bobine. Les spectres sont acquis en 5 minutes par la sommation de 428 signaux d'induction libre avec un angle d'impulsion de 45° et un temps de répétition de 0,7sec. La largeur spectrale est de 6000Hz et la taille de mémoire de 2K. Avant la transformation de Fourier, le signal d'induction libre est multiplié par une fonction exponentielle générant un élargissement des raies de 20Hz.

### 1.4.1. Analyse des données de SRM du P-31

Les signaux observés sur le spectre de RMN du P-31 sont ceux du phosphate inorganique, de la phosphocréatine et des 3 phosphores de l'ATP. Sur certains spectres on peut observer un signal attribué aux phosphomonoesters (sucres phosphates). Les aires et les positions des résonances sont déterminées en utilisant le programme NMR1 (New Methods Research Syracuse , NY) sur une station de traitement IBM Risc System 6000.

### 1.4.2. Quantification des dérivés phosphorés

Les aires mesurées sont corrigées en tenant compte des facteurs de saturation déterminés en comparant les spectres acquis à des spectres complètement relaxés. Les aires des différents métabolites peuvent alors être converties en concentrations en se rapportant à l'aire de l'ATPβ, considérant une concentration cytosolique de 11,6mM pour l'ATP (déterminée par chromatographie liquide haute pression).

### 1.4.3. Mesure du pH intracellulaire

La valeur du pH est calculée à partir du déplacement chimique du phosphate inorganique, relativement à la résonance de la phosphocréatine. En effet, sa position sur le spectre est indépendante du pH, permettant de relier le déplacement chimique du signal au pH grâce à une courbe de titration.

### 1.5. Analyses biochimiques

### 1.5.1. Dosages dans les effluats de coeur

### Dosage de la créatine kinase

La créatine kinase dosée dans les effluats est un bon indicateur de l'intégrité cellulaire, la fuite en créatine kinase étant proportionnelle à l'intensité de la lyse cellulaire. L'activité totale de la CK est mesurée suivant la méthode de Rosalki à 30°C en utilisant un kit sigma [Rosalki, S.B. An improved procedure for serum creatine phosphokinase determination. *J. Lab. Med.* 69, 696-705 (1967).] Un petit volume d'éffluat est congelé de manière à pouvoir doser ultérieurement, le phosphate et le lactate ainsi que les purines.

### Dosage du phosphate

Le phosphate est dosé par méthode colorimétrique [Ames, B.N. Assay of inorganic phosphates, total phosphate and phosphatases. *Methods in Enzymology*. **8**, 115-118(1966)]

### Dosage du lactate

Le lactate est dosé de manière enzymatique [Gutman, I. and Wahlefeld, A.W.L-(+)-.Lactase. Determination with lactate dehydrogenase and NAD. In « Methods of Enzymatic Analysis » (H.U. Bergmeymer), 3, 1464-1468. Acad Press NY, New Yord, 1974].

### Dosage des purines

Le dosage des purines est réalisé par CLHP en mode isocratique [Wynants, J., et al. Optimization of a HPLC method for the determination of nucleosides and their catabolites. Application to cat and rabbit heart perfusates. *J Chromatogr.* 386, 297-275 (1951)]. sur colonne Lichrosorb RP select B(45) à l'aide d'un système chromatographique (LKB, Bromma, Suède) comportant une pompe (modèle 2150), un détecteur à longueur d'onde variable (modèle 2151), un contrôleur (modèle 2152) ainsi qu'une vanne d'injection (Rheodyne 7125). Les données sont acquises en utilisant un programme d'intégration KONTRON pour PC (Kontron Instruments Munich, FRG). Les purines mises en évidence sont l'adénosine, l'inosine, l'allopurinol, la xanthine et l'hypoxanthine.

### 1.5.2. Dosages dans les coeurs congelés

A la fin de chaque expérience, les coeurs sont rapidement plongés dans l'azote liquide (freeze-clamping). Les coeurs sont ensuite placés au congélateur à -80°C pour des analyses métaboliques ultérieures et la détermination de leur contenu en eau.

### Détermination du contenu en eau

Des fragments de coeur sont décongelés, pesés, soumis à dessiccation pendant 48 heures puis repesés après cette perte d'eau. Cette technique permet d'évaluer le pourcentage d'eau dans les tissus et d'en déduire l'importance de l'oedème après reperfusion.

### Dosage de la créatine kinase

Lors des dosages, environ 30mg de coeur sont décongelés et homogénéisés dans un tampon de potassium phosphate, le dosage de la créatine kinase est ensuite réalisé par la même méthode enzymatique que dans les effluats. Les activités enzymatiques sont exprimées en unités internationales (UI) par milligramme de protéines cardiaques mesurées suivant la méthode de Lowry et Coll [Lowry, O.H., et al., R.J. Protein measurement with the Folin phenol reagent. *J. Biol. Chem* **193**, 265-275 (1951)].

### Dosage de la lactate déshydrogénase (LDH)

L'activité de la LDH est mesurée par la méthode de Bernstein et Everse [Bernstein, L.K. and Everse, J. Determination of the isoenzyme levels of lactate deshydrogenase. *Methods Enzymol.* **41**, 47-52 (1975)] dans les coeurs traités par un tampon potassium phosphate.

### Dosage des nucléotides par chromatographie liquide haute pression (CLHP).

Les nucléotides sont dosés dans les extraits perchloriques des coeurs congelés à la fin de l'expérience. Le dosage des nucléotides est réalisé par CLHP en mode gradient sur colonne Lichrospher RP-18 sur le même système chromatographique que pour le dosage des purines. Le contenu total en nucléotides adényliques (ATP+ADP+AMP= TAN) est présenté dans les résultats.

### Dosage des acides aminés par chromatographie liquide haute pression (CLHP).

Les acides aminés libres sont mesurés dans les extraits perchloriques en utilisant la méthode Pico-Tag (Waters) fondée sur les résultats de Cohen et al [Cohen, S.A., etal., PITC derivatives in amino acid analysis. *Nature.* **320**, 769-770 (1986)].

### EXEMPLE 2 : TRANSPLANTATION CARDIAQUE HÉTÉROTOPIQUE ABDOMINALE CHEZ LE RAT LEWIS

Des rats mâles Lewis de 300 à 375g sont utilisés comme donneurs et receveurs. Tous les animaux reçoivent des soins et sont maintenus dans un environnement conforme aux législations réglementant l'expérimentation animale. L'anesthésie est induite et maintenue à l'éther. La transplantation se déroule selon une procédure propre, non stérile.

### Préparation du donneur

Après anesthésie, une laparotomie médiane xyphopubienne est réalisée et l'aorte et la veine cave sont contrôlées à l'étage sous rénal. 300 UI d'héparine sont injectées dans la veine cave inférieure, un long cathéter est introduit dans l'aorte de façon rétrograde et l'arrêt cardiaque est induit par injection de 40ml de liquide de cardioplégie. Le thorax est ensuite abordé par un volet costal antérieur. 10ml supplémentaires de liquide de cardioplégie sont injectés à travers la veine cave inférieure sus-diaphragmatique, afin de purger les cavités cardiaques. Les veines caves inférieures, supérieures droite et gauche sont liées. L'artère pulmonaire est sectionnée prés de sa bifurcation et l'aorte, au pied du TABC. Enfin, les veines pulmonaires sont immédiatement immergées dans le liquide de cardioplégie à 4°C.

### Préparation du receveur

Après anesthésie, une laparotomie médiane xyphopubienne est réalisée. L'aorte et la veine cave sont contrôlées et exposées en sous-rénal. Après électrocoagulation des artères et des veines lombaires, les vaisseaux sont clampés par un double clamp approximateur.

Le greffon est placé transversalement à gauche de l'aorte du receveur, dans la cavité abdominale, l'hypothermie locale est maintenue à l'aide d'un glaçon. La suture aortique est menée en premier (anastomose termino-latérale entre l'aorte du donneur et l'aorte du receveur au monofil de polyamide 10/0-aiguille BV70, puis l'anastomose entre l'artère pulmonaire du donneur et la veine cave du receveur est conduite de la même manière). Un déclamplage progressif des vaisseaux en aval puis en amont est ensuite réalisé pendant que le coeur est réchauffé à l'aide de sérum à 37°C. Après vérification de l'hémostase, l'abdomen est ensuite refermé en 2 plans par un surjet.

A la 24ème heure, l'animal est à nouveau anesthésié pour une 2ème laparotomie. L'aspect fonctionnel du greffon est noté. Le greffon est excisé, puis canulé et perfusé au krebs pour étude hémodynamique et métabolique par spectroscopie de résonance magnétique du phosphore-31. L'animal est ensuite sacrifié.

### EXEMPLE COMPARATIF 3

Protocole 1 : Evaluation hémodynamique et métabolique par spectroscopie de résonance magnétique du P-31 sur des coeurs soumis à une ischémie totale de 12 heures, après un arrêt cardioplégique sur une préparation de coeur isolé, perfusé.

Dans ce protocole, 4 solutions cardioplégiques ont été comparées, la solution UW, la solution de Broussais, la solution de St Thomas et la solution CRMBM. Les résultats montrent une meilleure préservation du métabolisme et de l'intégrité cellulaire au cours de l'ischémie et de la reperfusion après une ischémie de 12 heures avec la solution CRMBM, comme cela a déjà été observé dans un protocole de 6 heures d'ischémie (non montré). La récupération fonctionnelle est très significativement améliorée avec cette solution après une ischémie de 12 heures puisqu'elle atteint 50 % dans ce groupe alors qu'elle est pratiquement nulle avec les autres solutions.

Protocole 2 : Evaluation métabolique et hémodynamique sur le modèle de la transplantation hétérotopique en position abdominale chez le rat.

Le modèle de la transplantation hétérotopique permet de réaliser une reperfusion physiologique (au sang, déclampage progressif, assistance cardiaque prolongée). D'autre part, il permet d'évaluer la récupération métabolique et fonctionnelle à long terme.

Les coeurs sont divisés en 2 groupes dans cette étude :
Groupe A (contrôle) : la transplantation est effectuée immédiatement après le prélèvement (la durée d'ischémie est de 40 mn environ).
Groupe B : le coeur prélevé sur le donneur subit une ischémie froide de 5h20 avant transplantation (ischémie totale de 6 heures en comptant la durée de la réalisation opératoire de la transplantation).

Après implantation du coeur, l'animal receveur est refermé et le coeur est examiné après une période de 24 H. Une étude du coeur est d'abord réalisée *in situ* sur l'animal entier par SRM du P-31 sur le système Biospec en utilisant une bobine de surface double accord ¹H-³¹P de 1,5 cm de diamètre. Son aspect morphologique est également examiné puis, comme le coeur ne travaille pas dans ce modèle, le coeur est excisé et une évaluation hémodynamique est réalisée sur un modèle de coeur isovolumique avec évaluation du métabolisme énergétique par spectroscopie de résonance magnétique (étude sur le spectromètre Bruker-Nicolet WP2000 couplé à un aimant vertical de 4,7T). Des dosages biochimiques sont également réalisés dans les effluats et dans les coeurs congelés à la fin des expériences.

Les coeurs transplantés dans les 2 groupes ont tous repris des contractions régulières lors du déclampage. Après 24 heures les coeurs examinés *in situ* par SRM présentent des niveaux similaires en métabolites énergétiques quelle que soit la durée de l'ischémie. De même les coeurs explantés et perfusés sont caractérisés par des indices métaboliques et fonctionnels similaires dans les deux groupes.

Les résultats obtenus sur le modèle de la transplantation hétérotopique chez le rat montrent que la solution mise au point apporte une bonne protection sur ce modèle et valident les résultats acquis sur le modèle du coeur isolé, perfusé.

Protocole 3 : amélioration de la solution CRMBM par l'ajout de L-arginine : étude hémodynamique et métabolique sur le modèle de coeur isolé et perfusé.

L'apport de L-arginine à la solution de préservation a été étudié sur le modèle du coeur isolé, perfusé décrit précédemment, soumis à 8 H d'ischémie. Les résultats montrent une amélioration de la récupération fonctionnelle post-ischémique et un pool plus élevé en acides aminés au cours de la reperfusion.

Protocole 4 : comparaison de la solution CRMBM avec la solution Celsior récemment brevetée : étude hémodynamique et métabolique sur le modèle de coeur isolé et perfusé.

L'étude réalisée sur le coeur isolé, perfusé, soumis à 8 heures d'ischémie démontre l'obtention d'une meilleure protection de l'intégrité membranaire avec la solution CRMBM, ainsi que l'atteste en particulier une perte réduite de la créatine kinase au cours de la reperfusion.

### EXEMPLE 3: EFFETS METABOLIQUES ET FONCTIONNELS DE SOLUTIONS CARDIOPLEGIQUES A FAIBLE CONCENTRATION EN POTASSIUM POUR LA PRESERVATION A LONG TERME DU COEUR

Dans le but de minimiser les effets secondaires liés au potassium, deux concentrations en potassium (4mM et 10mM) ont été évaluées dans la solution de préservation.

La composition d'une solution de l'invention contenant 4 mM de potassium est telle que celle décrite ci-dessus, et comprend notamment KCl (2 mM) et KH₂PO₄ (2 mM).

La composition d'une solution contenant 10 mM de potassium est telle que celle décrite ci-dessus, et comprend notamment KCl (2 mM) et KH₂PO₄ (8 mM).

La spectrométrie de résonance magnétique du phosphore-31 est utilisée durant tout le protocole expérimental afin de suivre le métabolisme énergétique et le pH intracellulaire.

La récupération fonctionnelle est mesurée avant et après la période ischémique.

Des dosages biochimiques sont également réalisés dans les effluats coronaires et dans les coeurs congelés à la fin de la reperfusion afin de déterminer le dommage membranaire.

Les résultats obtenus permettent de démontrer (cf figures 15, 16A et 16B) que la diminution de la concentration en potassium dans la solution çardioplégique CRMBM améliorait la récupération fonctionnelle et limitait le dommage membranaire de coeurs de rats soumis à une ischémie hypothermique de 8 heures et reperfusés tout en préservant les composés riches en énergie.

### EXEMPLE 4: EFFET DE L'AJOUT DE L-ARGININE SUR LES VARIATIONS DES CONCENTRATIONS EN ACIDES AMINES ET NUCLEOTIDES ADENYLIQUES AU COURS D'UNE ISCHEMIE A LONG TERME ET DE LA REPERFUSION DE COEUR DE RAT : EFFET DE L'AJOUT DE LA L-ARGININE.

Différents travaux effectués ces dernières années ont montré qu'une séquence d'ischémie et reperfusion entraînait des altérations des contenus myocardiques en acides aminés dans des modèles expérimentaux animaux et dans des coeurs humains.

L'influence de la présence de L-arginine dans la solution cardioplégique de l'invention a été évaluée sur les concentrations intracellulaires d'acides aminés, de nucléotides et de PCr en fin d'ischémie et en fin de reperfusion (cf figures 17A, 17B, 18A et 18B).

Les déterminations des métabolites ont été réalisées par chromatographie liquide haute performance (HPLC).

L'ajout de L-arginine dans la solution limite la déplétion du pool des acides aminés lors de la reperfusion et améliore la récupération fonctionnelle post-ischémique sans affecter toutefois le contenu en nucléotides adényliques.

### EXEMPLE 5: EFFET DE L'AJOUT DE L-ARGININE SUR LA FONCTION DE L'ENDOTHELIUM ET DU MUSCLE LISSE VASCULAIRE.

La 5-hydroxytryptamine (5-HT) et la papaverine ont été utilisées pour analyser les variations des réponses vasodilatatrices respectivement dépendantes et indépendantes de l'endothélium lors des périodes pré- et post-ischémique.

Avant l'ischémie, le débit coronaire est mesuré dans chaque groupe durant la perfusion avec le tampon normal et durant la perfusion de 5-HT et de papaverine. Après l'ischémie, le pourcentage de récupération de la réponse pré-ischémique du débit coronaire à la perfusion de 5-HT et de papaverine est calculé.

Dans le groupe contrôle, les réponses vasodilatatrices au 5-HT et à la papaverine sont complètement perdues et on observe une vasoconstriction avec les deux drogues.

Par contre, dans le groupe recevant la L-arginine, la réponse vasodilatatrice dépendante de l'endothélium est perdue tandis que l'on observe une légère récupération de la réponse pré-ischémique à la papaverine, indiquant que le muscle vasculaire lisse est moins altéré que l'endothélium coronaire.
(cf figures 19A et 19B)

### EXEMPLE 6: RECUPERATION FONCTIONNELLE ET METABOLISME ENERGETIQUE AU COURS DE LA REPERFUSION A COURT TERME ET LONG TERME DANS LE MODELE DE TRANSPLANTATION HETEROTOPIQUE DE COEUR DE RAT.

Un modèle de transplantation hétérotopique de coeur de rat a été développé, travaillant où la fonction et le métabolisme peuvent être analysés simultanément. Sur ce modèle a ainsi été étudié la récupération fonctionnelle et métabolique après 1 heure et 24 heures de reperfusion après une ischémie de 3 heures, protégée par la solution de l'invention

L'étude a permis d'analyser la récupération à long terme du coeur, comparant 1 heure et 24 heures de reperfusion.

L'étude montre une amélioration de la récupération fonctionnelle après 24 heures de reperfusion, en comparaison à 1 heure de reperfusion, associée à une amélioration du métabolisme énergétique.

L'analyse des rapports des métabolites énergétiques (cf figure 20) suggère une régénération du pool intracellulaire d'ATP après 24 heures de reperfusion, en comparaison à 1 h de reperfusion, pouvant être associée à la resynthèse des précurseurs de l'ATP. En effet, lors de la phase précoce de reperfusion, il y a une perte des précurseurs métaboliques de l'ATP par la sortie de ces composés dans le milieu extracellulaire. Il est donc intéressant de voir si la cellule va être capable de resynthétiser ces précurseurs. Dans les conditions d'ischémie et de reperfusion de la présente invention, une récupération du métabolisme énergétique est obtenue (cf rapports de métabolites), qui suggère une resynthèse effective des précurseurs métaboliques de l'ATP.

L'effet de la L-arginine a également été étudié sur la reperfusion à court terme et long terme (cf figure 21). Les premiers résultats obtenus sur la récupération fonctionnelle du coeur montrent un effet bénéfique de la L-arginine aussi bien après 1 heure de reperfusion qu'après 24 heures de reperfusion.

## Revendications

1. Solution de perfusion et/ou de conservation et/ou de reperfusion lors de la transplantation d'organes, notamment de l'organe cardiaque, **caractérisée en ce qu'**elle contient les éléments suivants :
| | |
|---|---|
| K⁺ | à raison d'environ 4 à environ 7 mM, notamment environ 4 mM |
| Ca²⁺ | à raison d'environ 0,2 à environ 0,3 mM, notamment environ 0,25 mM |
| Mg²⁺ | à raison d'environ 13 à environ 16 mM, notamment environ 13 mM |
| glutamate | à raison d'environ 18 à environ 22 mM, notamment environ 20 mM |
| arginine | à raison d'environ 2 à environ 4 mM, notamment environ 3 mM |
| adénosine | à raison d'environ 0,5 à environ 1 mM, notamment environ 0,5 mM |

2. Solution selon la revendication 1, **caractérisée en ce qu'**elle contient au moins un agent imperméant choisi par ordre de préférence parmi l'acide lactobionique, le mannitol et le raffinose

3. Solution selon l'une quelconque des revendications 1 à 2, **caractérisée en ce qu'**elle contient au moins un agent piégeur de radicaux libres, notamment choisi parmi le glutathion (forme réduite), l'allopurinol, ou le mannitol.

4. Solution selon l'une quelconque des revendications 1 à 3, **caractérisée en ce qu'**elle contient les éléments suivants :
| | |
|---|---|
| K⁺ | à raison d'environ 4 à environ 7 mM, notamment environ 4 mM |
| Ca²⁺ | à raison d'environ 0,2 à environ 0,3 mM, notamment environ 0,25 mM |
| Mg²⁺ | à raison d'environ 13 à environ 16 mM, notamment environ 13 mM |
| glutamate | à raison d'environ 18 à environ 22 mM, notamment environ 20 mM |
| arginine | à raison d'environ 2 à environ 4 mM, notamment environ 3 mM |
| adénosine | à raison d'environ 0,5 à environ 1 mM, notamment environ 0,5 mM |
- au moins un agent imperméant,
- au moins un agent piégeur de radicaux libres
| | |
|---|---|
| Osmolarité | 340 mM |
| pH | 7,4 |

5. Solution selon l'une quelconque des revendications 1 à 4, **caractérisée en ce qu'**elle contient les éléments suivants :
| | |
|---|---|
| K⁺ | d'environ 4 à environ 7 mM |
| Ca²⁺ | d'environ 0,2 à environ 0,3 mM |
| Na⁺ | d'environ 108 à environ 132 mM |
| Mg²⁺ | d'environ 13 à environ 16 mM |
| glutamate | d'environ 18 à environ 22 mM |
| arginine | d'environ 2 à environ 4 mM |
| adénosine | d'environ 0,5 à environ 1 mM |
| mannitol | d'environ 27 à environ 33 mM |
| allopurinol | d'environ 0,9 à environ 1,1 mM |
| glutathion (forme réduite) | d'environ 2,7 à environ 3,3 mM |
| raffinose | d'environ 25 à environ 35 mM |
| acide lactobionique | d'environ 80 à environ 120 mM |
| pH | d'environ 7,2 à environ 7,4 |
| osmolarité | d'environ 330 à environ 360 mOsm |

6. Solution selon la revendication 5, dans laquelle Na⁺ est sous forme NaOH, K⁺ est sous forme KCl et KH₂ PO₄, avantageusement à raison d'environ 2 à environ 3,5 mM KCl et d'environ 2 à environ 3,5 mM KH₂ PO₄, Ca²⁺ est sous forme de Ca Cl₂, 2H₂O et Mg²⁺ est sous forme MgCl₂, 6H₂O.

7. Utilisation d'une solution selon l'une quelconque des revendications 1 à 6 pour la conservation d'organes, notamment de l'organe cardiaque pendant une durée d'au moins 12 à 15 heures et notamment pendant au moins 12 heures.

8. Utilisation selon la revendication 7 d'une solution selon l'une quelconque des revendications 1 à 6, pour maintenir l'intégrité cellulaire et métabolique de l'organe après ischémie, cette intégrité pouvant être détectée par mesure des activités de l'un au moins des enzymes suivantes : lactate déshydrogénase, créatine kinase et/ou par mesure de l'un au moins des métabolites suivants : purines, nucléotides notamment nucléotides adényliques, inosine monophosphate, adénosine triphosphate, acides aminés, phosphate inorganique, lactate, phosphocréatine.

9. Utilisation selon la revendication 7, d'une solution selon l'une quelconque des revendications 1 à 6 pour assurer la récupération fonctionnelle post ischémique, cette récupération fonctionnelle pouvant être mesurée par la mesure d'un au moins des paramètres hémodynamiques suivants : le débit coronaire, la pression développée, la fréquence cardiaque, et la pression diastolique.

## Patentansprüche

1. Perfusions- und/oder Konservierungs- und/oder Reperfusionslösung zur Organtransplantation, insbesondere des Herzorgans, **dadurch gekennzeichnet, dass** sie die folgenden Elemente enthält:
| | |
|---|---|
| K⁺ | im Bereich von ungefähr 4 bis ungefähr 7 mM, insbesondere ungefähr 4 mM |
| Ca²⁺ | im Bereich von ungefähr 0,2 bis ungefähr 0,3 mM, insbesondere ungefähr 0,25 mM |
| Mg²⁺ | im Bereich von ungefähr 13 bis ungefähr 16 mM, insbesondere ungefähr 13 mM |
| Glutamat | im Bereich von ungefähr 18 bis ungefähr 22 mM, insbesondere ungefähr 20 mM |
| Arginin | im Bereich von ungefähr 2 bis ungefähr 4 mM, insbesondere ungefähr 3 mM |
| Adenosin | im Bereich von ungefähr 0,5 bis ungefähr 1 mM, insbesondere ungefähr 0,5 mM |

2. Lösung gemäß Anspruch 1, **dadurch gekennzeichnet, dass** sie wenigstens ein undurchgängiges Mittel enthält, dass in der Reichenfolge der Präferenz unter der Lactobionsäure, dem Mannitol und der Raffinose ausgewählt ist.

3. Lösung gemäß einem der Ansprüche 1 bis 2, **dadurch gekennzeichnet, dass** diese wenigstens ein Abfangmittel für freie Radikale enthält, insbesondere ausgewählt unter dem Glutathion (reduzierte Form), dem Allopurinol oder dem Mannitol.

4. Lösung gemäß einem der Ansprüche 1 bis 3, **dadurch gekennzeichnet, dass** diese die folgenden Elemente enthält:
| | |
|---|---|
| K⁺ | im Bereich von ungefähr 4 bis ungefähr 7 mM, insbesondere ungefähr 4 mM |
| Ca²⁺ | im Bereich von ungefähr 0,2 bis ungefähr 0,3mM, insbesondere ungefähr 0,25 mM |
| Mg²⁺ | im Bereich von ungefähr 13 bis ungefähr 16mM, insbesondere ungefähr 13 mM |
| Glutamat | im Bereich von ungefähr 18 bis ungefähr 22mM, insbesondere ungefähr 20 mM |
| Arginin | im Bereich von ungefähr 2 bis ungefähr 4 mM, insbesondere ungefähr 3 mM |
| Adenosin | im Bereich von ungefähr 0,5 bis ungefähr 1 mM, insbesondere ungefähr 0,5 mM |
- wenigstens ein undurchgängiges Mittel,
- wenigstens ein Abfangmittel für freie Radikale
| | |
|---|---|
| Osmotischer Wert | 340 mM |
| pH | 7,4 |

5. Lösung gemäß einem der Ansprüche 1 bis 4, **dadurch gekennzeichnet, dass** diese die folgenden Elemente enthält:
| | |
|---|---|
| K⁺ | von ungefähr 4 bis ungefähr 7 mM |
| Ca²⁺ | von ungefähr 0,2 bis ungefähr 0,3 mM |
| Na⁺ | von ungefähr 108 bis ungefähr 132 mM |
| Mg²⁺ | von ungefähr 13 bis ungefähr 16 mM |
| Glutamat | von ungefähr 18 bis ungefähr 22 mM |
| Arginin | von ungefähr 2 bis ungefähr 4 mM |
| Adenosin | von ungefähr 0,5 bis ungefähr 1 mM |
| Mannitol | von ungefähr 27 bis ungefähr 33 mM |
| Allopurinol | von ungefähr 0,9 bis ungefähr 1,1 mM |
| Glutathion (reduzierte Form) | von ungefähr 2,7 bis ungefähr 3,3 mM |
| Raffinose | von ungefähr 25 bis ungefähr 35mM |
| Lactobionsäure | von ungefähr 80 bis ungefähr 120 mM |
| pH | von ungefähr 7,2 bis ungefähr 7,4 |
| Osmotischer Wert | von ungefähr 330 bis ungefähr 360 mOsm |

6. Lösung gemäß Anspruch 5, in welcher Na⁺ in der Form NaOH ist, K⁺ in der Form von KCL und KH₂PO₄ ist, vorteilhafterweise in einem Bereich von ungefähr 2 bis ungefähr 3,5 mM KCL und von ungefähr 2 bis ungefähr 3,5 mM KH₂PO₄, Ca²⁺ in der Form von CaCl₂, 2H₂O ist und Mg²⁺ in der Form von MgCl₂, 6H₂O ist.

7. Verwendung einer Lösung gemäß einem der Ansprüche 1 bis 6 für die Konservierung von Organen, insbesondere des Herzorgans während einer Dauer von ungefähr 12 bis 15 Stunden und insbesondere während mindestens 12 Stunden.

8. Verwendung gemäß Anspruch 7 einer Lösung gemäß einem der Ansprüche 1 bis 6, um die Zell- und Stoffwechselintegrität des Organs nach Ischämie beizubehalten, wobei diese Integrität durch Messung der Aktivitäten von wenigstens einem der folgenden Enzyme ermittelt werden kann: Laktatdehydrogenase, Kreatinkinase und/oder durch Messung von wenigstens einem der folgenden Metaboliten: Purine, Nukleotide, insbesondere adenylinische Nukleotide, Inosinmonophosphat, Adenosintriphosphat, Aminosäuren, anorganisches Phosphat, Laktat, Phosphokreatin.

9. Verwendung gemäß Anspruch 7 einer Lösung gemäß einem der Ansprüche 1 bis 6, um die post-ischämische funktionelle Erholung sicherzustellen, wobei diese funktionelle Erholung durch die Messung von wenigstens den folgenden hämodynamischen Parametern gemessen werden kann: der Koronar-Durchsatz, der entwickelte Druck, die Herzfrequenz und der diastolische Druck.

## Claims

1. Solution for perfusion and/or preservation and/or re-perfusion during organ transplantation, especially the heart, **characterised in that** it contains the following elements:
| | |
|---|---|
| K⁺ | at a rate from about 4 to about 7 mM, especially about 4 mM |
| Ca²⁺ | at a rate from about 0.2 to about 0.3 mM, especially about 0.25 mM |
| Mg²⁺ | at a rate from about 13 to about 16 mM, especially about 13 mM |
| glutamate | at a rate from about 18 to about 22 mM, especially about 20 mM |
| arginine | at a rate from about 2 to about 4 mM, especially about 3 mM |
| adenosine | at a rate from about 0.5 to about 1 mM, especially about 0.5 mM |

2. Solution according to claim 1, **characterised in that** it contains at least one water resistant agent chosen by order of preference from lactobionic acid, mannitol and raffinose.

3. Solution according to any of claims 1 to 2, **characterised in that** it contains at least one agent trapping free radicals, chosen notably from glutathion (reduced form), allopurinol, or mannitol.

4. Solution according to any of claims 1 to 3, **characterised in that** it contains the following elements:
| | |
|---|---|
| K⁺ | at a rate from about 4 to about 7 mM, especially about 4 mM |
| Ca²⁺ | at a rate from about 0.2 to about 0.3 mM, especially about 0.25 mM |
| Mg²⁺ | at a rate from about 13 to about 16 mM, especially about 13 mM |
| glutamate | at a rate from about 18 to about 22 mM, especially about 20 mM |
| arginine | at a rate from about 2 to about 4 mM, especially about 3 mM |
| adenosine | at a rate from about 0.5 to about 1 mM, especially about 0.5 mM |
- at least one water resistant agent,
- at least one agent trapping free radicals
| | |
|---|---|
| Osmolarity | 340 mM |
| pH | 7.4 |

5. Solution according to any of claims 1 to 4, **characterised in that** it contains the following elements:
| | |
|---|---|
| K⁺ | from about 4 to about 7 mM, |
| Ca²⁺ | from about 0.2 to about 0.3 mM, |
| Na⁺ | from about 108 to about 132 mM |
| Mg²⁺ | from about 13 to about 16 mM, |
| glutamate | from about 18 to about 22 mM, |
| arginine | from about 2 to about 4 mM, |
| adenosine | from about 0.5 to about 1 mM |
| mannitol | from about 27 to about 33 mM |
| allopurinol | from about 0.9 to about 1.1 mM |
| glutathion (reduced form) | from about 2.7 to about 3.3 mM |
| raffinose | from about 25 to about 35 mM |
| lactobionic acid | from about 80 to about 120 mM |
| pH | from about 7.2 to about 7.4 |
| osmolarity | from about 330 to about 360 mOsm |

6. Solution according to claim 5, in which Na⁺ is in the form NaOH, K⁺ is in the form KCl and KH₂PO₄, favourably at a rate from about 2 to about 3.5 mM KCI and about 2 to about 3.5 mM KH₂PO₄, Ca²⁺ is in the form of CaCl₂, 2H₂O and Mg²⁺ is in the form MgCl₂, 6H₂O.

7. Use of a solution according to any of claims 1 to 6 for the preservation of organs, notably the heart for a duration of at least 12 to 15 hours and especially for at least 12 hours.

8. Use according to claim 7 of a solution according to any of claims 1 to 6, to maintain the cellular and metabolic integrity of the organ after ischemia, this integrity being able to be detected by measurement of the activities of at least one of the following enzymes: lactate dehydrogenase, creatine kinase and/or by measurement of at least one of the following metabolites: purines, nucleotides notably adenylic nucleotides, inosine monophosphate, adenosine triphosphate, amino acids, inorganic phosphate, lactate, phosphocreatine.

9. Use according to claim 7, of a solution according to any of claims 1 to 6 to ensure post ischemic functional recovery, this functional recovery able to be measured by the measurement of at least one of the following hemodynamic parameters: coronary flow, developed pressure, cardiac frequency, and diastolic pressure.
